# EUROPEAN PATENT APPLICATION

(11) **EP 1 886 696 A1**
(43) Date of publication of application: **13.02.2008**
(21) Application number: 06291261.3
(22) Date of filing: 03.08.2006
(51) Int. Cl.: A61K 47/48

(54) **Conjugates of antithrombin binding oligosaccharide derivatives and therapeutic proteins**

(71) Applicant: Endotis Pharma, 59120 Loos (FR)
(72) Inventor: Gauchet, Cécile, 75011 Paris (FR); Huet, Thierry, 94130 Nogent Sur Marne (FR); Lucidi, Bruno, 1180 Bruxelles (BE); Petitou, Maurice, 75645 Paris Cedex 13 (FR); Querolle, Olivier, 91300 Massy (FR)
(74) Representative: Howard, Paul Nicholas

(57) **Abstract**

The present invention is concerned with protein or glycoprotein derivatives, their intermediates, uses thereof and processes for their production. In particular, the present invention relates to improving the pharmacokinetic profile of drugs that contain proteins.

## Description

All documents cited herein are incorporated by reference in their entirety.

### TECHNICAL FIELD

The present invention is concerned with protein or glycoprotein derivatives, their intermediates, uses thereof and processes for their production. In particular, the present invention relates to improving the pharmacokinetic profile of drugs that contain proteins.

### BACKGROUND ART

Proteins often suffer from poor distribution properties and do not have particularly long half-lives *in vivo.* Many drugs contain proteins and these drugs have short half-lives in the blood stream and a low overall clearance rate. Accordingly, frequent administration is required in order to combat the poor pharmacokinetic profiles of such drugs.

It is known in the art that proteins have been conjugated to small molecules in an attempt to improve their *in vivo* characteristics, such as: improving their physical and thermal stability, protecting them against degradation by enzymes, enhancing their solubility, prolonging their half-life and, in some cases, reducing their immunogenicity. For example, in an attempt to increase the pharmacokinetic profile of low molecular weight proteins (i.e. proteins less than 20 kDa), polyethylene glycol (PEG) has been conjugated to proteins. PEG can increase the hydrodynamic volume of a protein; reduce an immune response to a protein; and prevent attacks on a protein by proteases. In effect, using PEG in protein conjugation masks the presence of the protein *in vivo* to a certain extent. Typical conjugation techniques are described in Drug Discovery Today (DDT), 2005, 10(21), 1451-1458 and Biomaterials, 2001, 22, 405-417, for example.

There are, however, problems associated with directly conjugating PEG to proteins, for example, it can be difficult to selectively conjugate PEG directly to proteins. Coupling reactions between PEG and a protein typically generate multiple PEGylated isoforms with differing chemical and pharmaceutical properties (i.e. each isoform has a different pharmacokinetic profile). In addition, if PEG is conjugated with high molecular weight proteins (i.e. proteins greater than 20 kDa) it can be difficult to excrete the compounds from the body and the PEG-protein complex congregates in the liver.

There therefore remains a need for a method of improving the pharmacokinetic profile of proteins and, in particular, drugs that contain proteins. Ideally, the proteins or drugs that contain proteins should exist in either a single isoform or a mixture of isoforms that can be easily separated. The present invention aims to increase the pharmacokinetic profile of proteins *in vivo* and, in particular, increase the half-lives of proteins *in vivo.*

Although it is known in the art for oligosaccharides to be conjugated to small molecules, such an approach has not been attempted with proteins in order to increase their pharmacokinetic profile. An example of an oligosaccharide that has been conjugated to a small molecule can be seen in US2003/114361. Additionally, an example of an oligosaccharide that has been conjugated to a protein can be seen in WO 2005/000862, wherein an oligosaccharide is conjugated to a protein *via* a selenenylsulfide bond.

### DISCLOSURE OF THE INVENTION

According to one aspect of the present invention, there is provided a compound comprising a protein conjugated to an oligosaccharide, *via* a linker, wherein the oligosaccharide is capable of binding to antithrombin (AT). Antithrombin has been previously known and referred to in the art as AT-III. The AT-binding oligosaccharide is preferably a pentasaccharide.

In another aspect of the present invention, there is provided use of an AT-binding oligosaccharide for conjugation to a protein *via* a linker in a method of increasing the biological half-life of the protein.

In another aspect of the invention, there is provided a compound comprising an AT-binding oligosaccharide as defined in the present invention conjugated to a linker as defined in the present invention, or a salt, solvate or prodrug thereof.

In another aspect of the invention, there is provided use of a compound comprising an AT-binding oligosaccharide as defined in the present invention conjugated to a linker as defined in the present invention, or a salt, solvate or prodrug thereof, for conjugation to a protein in a method of increasing the biological half-life of the protein.

### OLIGOSACCHARIDE

The present inventors have found that oligosaccharides that bind to AT can improve the pharmacokinetic profile of a conjugated protein to approximately that of the oligosaccharide (i.e. approximately 5 to 50 times).

Although, as mentioned above, any oligosaccharide capable of binding to antithrombin (AT) can be used, the AT-binding oligosaccharides of the present invention are preferably of Formula (I) below: 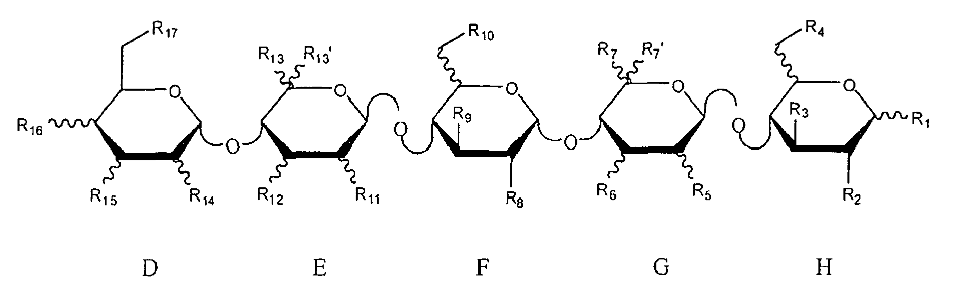 wherein:
R₁ is selected from the group consisting of: O-acyl, O-alkyl, O-alkenyl, O-alkynyl, O-alkylaryl, O-aryl and a monosaccharide unit of formula 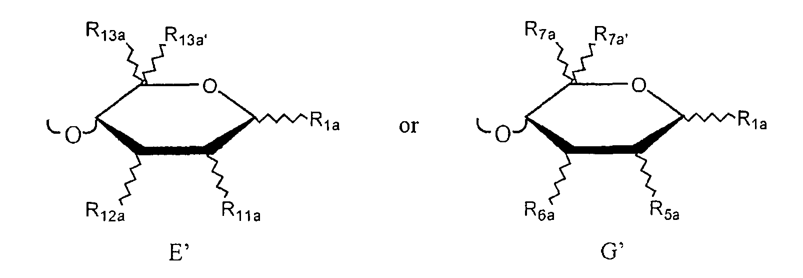 wherein R₁ₐ is selected from the group consisting of: O-acyl, O-alkyl, O-alkenyl, O-alkynyl, O-alkylaryl and O-aryl;
R₁₆ is selected from the group consisting of: OSO₃H, OH, O-acyl, O-alkyl, O-alkenyl, O-alkynyl, O-alkylaryl, O-aryl and a monosaccharide unit of formula 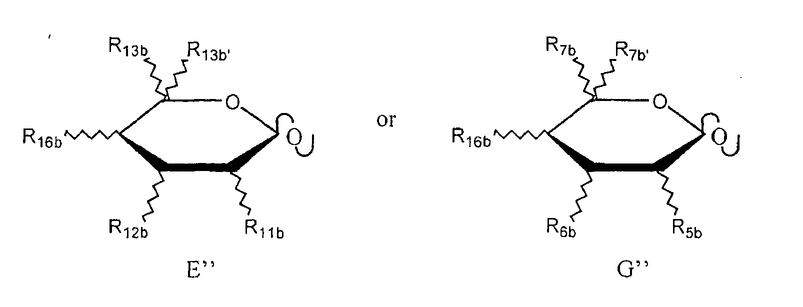 wherein R_{16b} is selected from the group consisting of: OSO₃H, OH, O-acyl, O-alkyl, O-alkenyl, O-alkynyl, O-alkylaryl and O-aryl;
R₂ and R₈ are each independently selected from the group consisting of: OSO₃H and NHSO₃H;
R₃, R₄, R₆, R₆ₐ, R_{6b}, R₁₀, R₁₁, R₁₁ₐ, R_{11b}, R₁₂, R₁₂ₐ and R_{12b} are each independently selected from the group consisting of: OSO₃H, O-acyl, O-alkyl, O-alkenyl, O-alkynyl, O-alkylaryl and O-aryl;
R₅, R₅ₐ and R_{5b} are each independently selected from the group consisting of: OSO₃H, O-acyl, O-alkyl, O-alkenyl, O-alkynyl, O-alkylaryl and O-aryl;
R₇, R₇ₐ and R_{7b} are COOH;
R_{7'}, R_{7a'} and R_{7b'} are H;
alternatively, R₅ together with R_{7'}, R₅ₐ, together with R_{7a'}, and R_{5b} together with R_{7b'} may each independently be selected from the group consisting of: O-CH₂ and O-CH₂CH₂ whereby, together with the carbon atoms to which they are attached, they form a heterocyclic ring;
R₉ and R₁₇ are each OSO₃H;
R₁₃, R₁₃ₐ and R_{13b} are each COOH;
R_{13'}, R_{13a'} and R_{13b'} are each independently selected from the group consisting of: H and alkyl;
R₁₄ is selected from the group consisting of: OSO₃H, NHSO₃H, OH, O-acyl, O-alkyl, O-alkenyl, O-alkynyl, O-alkylaryl and O-aryl; and
R₁₅ is selected from the group consisting of: OSO₃H, OH, O-acyl, O-alkyl, O-alkenyl, O-alkynyl, O-alkylaryl and O-aryl;
provided that at least one of the groups R₁, R₁ₐ, R₄, R₁₄, R₁₅, R₁₆ and R_{16b} is independently selected from the group consisting of: an amine, an oxygen atom and a carboxylic acid and is conjugated to the linker;
   or a salt, solvate or prodrug thereof.

In the present application, the groups -COOH, -OSO₃H and -NHSO₃H are represented in their acid form. In a preferred embodiment these groups are in their salt form, more preferably in their sodium salt form.

Advantageously, it has been found that a pentasaccharide is the optimal structural element that can be used for binding to AT and increasing the pharmacokinetic profile of proteins. Thus, it is preferable for the AT-binding oligosaccharide of the present invention to be a pentasaccharide.

In an alternative aspect of the present invention, R_{13'}, R_{13a'} and R_{13b'} are independently selected from the group consisting of: hydrogen and ethyl.

It will be appreciated that oligosaccharides and their component monosaccharide units can exist in a variety of stereochemical forms, which will be apparent to one skilled in the art. Positions of variable stereochemistry include those indicated with wavy lines. Except where specifically indicated, the present invention extends to all such stereochemical forms.

Preferably, the D, E, E', E", F and H monosaccharide units of the oligosaccharide have the D-gluco stereochemistry:

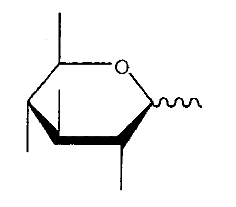

Additionally, it is preferred that the G, G' and G" monosaccharide units of the oligosaccharide have the L-ido stereochemistry:

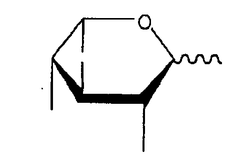

Optionally, R₅ together with R_{7'} may be selected from the group consisting of: O-CH₂ and O-CH₂CH₂ whereby, together with the carbon atoms to which they are attached, they form a heterocyclic ring.

The oligosaccharide is preferably conjugated to the linker through any one of the R₁, R₁ₐ, R₄, R₁₄, R₁₅, R₁₆ and R_{16b} positions of the oligosaccharide of Formula (I). More preferably, the oligosaccharide is conjugated to the linker through the R₁, R₄ or the R₁₆ position of the oligosaccharide, with the R₁₄ being particularly advantageous. It is preferred that the oligosaccharide is conjugated to the linker through either an amine functional group, which includes primary and secondary amines, an oxygen atom, or a carboxyl group. If an amine group is used, it is preferably a primary amine. Where reference is made in the present application to the oligosaccharide in unconjugated form, or the context so requires, it will be understood that the valency of functional group suitable for conjugation is completed with hydrogen atoms; for example, -O- and -NH- become -OH and -NH₂ respectively.

In a preferred aspect of the invention, the groups R₁, R₅, R₆, R₁₁, R₁₂, R₁₅, R₁₆ are each O-alkyl. More preferably, the groups R₁, R₅, R₆, R₁₁, R₁₂, R₁₅, R₁₆ are each O-CH₃.

In another preferred aspect of the invention, the groups R₂, R₃, R₄, R₈, R₉, R₁₀ and R₁₇ are each OSO₃H.

In another preferred aspect of the invention, the group R_{13'} is H.

In another preferred aspect of the invention, the group R₁₄ is NH.

Other examples of saccharides that are capable of inhibiting AT can be seen in European Patent EP 0 649 854, WO 98/03554 and WO 99/36443.

As mentioned above, AT-binding oligosaccharides conjugated to a protein *via* a linker can improve the pharmacokinetic profile of the protein. The compounds of the present invention increase the half-life of proteins to approximately that of the AT-binding oligosaccharide (typically about 120 hours in humans and much lower in animals). Preferably the conjugation of an AT-binding oligosaccharide to a protein improves the biological half-life of the protein by a factor of 2, preferably 5, more preferably 10, more preferably 20 and more preferably 50.

The coupling of oligosaccharides to proteins, typically, involves two steps: (a) derivatization of the saccharide; and (b) coupling of an intermediate with the protein. Consequently, a coupling step should, ideally, be fast and proceed under mild reaction conditions, which would ensure the biologically active components are not inactivated by, for example, denaturing the protein by exposing it to high temperatures.

### LINKER

The AT-binding oligosaccharides of the present invention are linked to a protein *via* a linker. Although it will be understood that the linker is a hydrocarbon chain optionally substituted with, or interrupted with, one or more heteroatoms, any suitable linker can be used. Preferably, the linker comprises a direct chain of from 6 to 250 atoms. More preferably, the lower limit of atoms in the direct chain of the linker is 6, 10, 15, 20, 25, 30, 35, 40, 45, 50, 60, 70, 80, 90, 100, 110 and 120; and the upper limit of atoms in the direct chain of the linker is 250, 245, 240, 235, 230, 225, 220, 215, 210, 200, 190, 180, 170, 160, 150, 140 and 130.

The linker is preferably conjugated to the AT-binding oligosaccharide through an amine, an amide, an ester or an ether bond. Additionally, it is preferred for the linker to be conjugated to the protein through an amine, an amine, an amide, an ether, an ester or a thioether bond.

It is preferable for the linker to further comprise at least one ethylene glycol monomer. More preferably, however, the linker comprises a PEG (polyethylene glycol) chain.

In another aspect of the present invention, it is preferable for the linker to further comprise at least one amino acid. More preferably, however, the linker comprises a poly-amino acid chain.

As discussed above, the oligosaccharides of the present invention bind to AT. The half-life of the AT-binding oligosaccharide is determined by its affinity for AT, which has been investigated in numerous pharmacokinetic studies *(e.g.* Vogel, G. M. T et. al.; Thromb. Haemostas, 1991, 65, 930). Therefore, the half-life of the AT-binding oligosaccharide-protein linker complex can be adjusted by altering the structure of the AT-binding oligosaccharide and linker.

It has been advantageously found that the linker used in the present invention may be of Formula II: 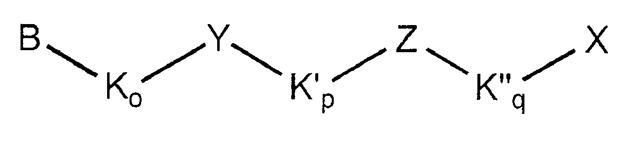 wherein:
B and X are both organic moieties, wherein the linker is conjugated to the AT-binding oligosaccharide *via* B and to the protein *via* X;
K, K', K" are independently selected from the group consisting of: an amino acid, an ethylene glycol monomer, alkyl, alkenyl and alkoxy;
o, p and q are integers independently selected from 0 to 40 and more preferably from 4 to 40, 5 to 35, 10 to 30, 15 to 25 and 12 to 20;
or Kₒ, K'ₚ and K''_{q} are independently a chain of amino acids that comprises from 2 to 40 amino acids, preferably from 3 to 10 amino acids, more preferably from 4 to 8 amino acids and more preferably from 5 to 7 amino acids;
Z is not present or is independently selected from the group consisting of: heterocyclyl and heteroaryl;
Y is not present or is independently selected from the group consisting of: a nitrogen atom, a carbon atom and a phosphorous atom optionally substituted with one or more groups of Formula IIa: 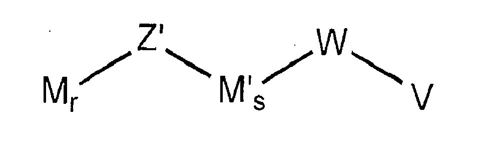 wherein M and M' are independently selected from the group consisting of: an amino acid, an ethylene glycol monomer, alkyl, alkenyl and alkoxy;
r and s are integers independently selected from 0 to 40 and more preferably from 4 to 40, 5 to 35, 10 to 30, 15 to 25 and 12 to 20;
or M and M' are independently a chain of amino acids that comprises from 2 to 40 amino acids, preferably from 3 to 10 amino acids, more preferably from 4 to 8 amino acids and more preferably from 5 to 7 amino acids;
Z' is not present or is independently selected from the group consisting of: heterocyclyl and heteroaryl;
W is an organic moiety through which the linker is conjugated to a protein;
V is a protein; and
wherein when any of K, K', K", M and M' are independently optionally substituted and/or interrupted with one or more heteroatoms.

Where Y is substituted with a group of Formula IIa, the group of Formula IIa is conjugated to a protein such that the AT-binding oligosaccharide can be conjugated to one, two or three proteins through a branched linker. Preferably, the proteins are the same proteins.

Where reference is made in the present application to the linker in unconjugated or partially conjugated form, or the context so requires, it will be understood that the valency of the functional group(s) suitable for conjugation is completed with hydrogen atoms.

Preferably, when any of K, K', K", M and M' are alkyl, they are independently optionally substituted and/or interrupted with one or more atoms independently selected from the group consisting of: oxygen, nitrogen and sulphur.

Advantageously, K, K', K", M and M' are each ethylene glycol monomers.

Preferably, Z and Z' are independently selected from the group consisting of: triazolyl and succinimidyl.

In a preferred aspect of the present invention, both Y and Z are not present.

In another preferred aspect of the present invention, p and q are both 0.

In another preferred aspect of the present invention, Z is independently selected from the group consisting of heterocyclyl and heteroaryl and Y is not present.

Preferably, B is selected from the group consisting of: alkyl, alkenyl, alkynyl, aryl, heteroaryl, heterocyclyl, aminoalkyl, O, R_{A}-O, R_{A}-O-R_{A}, COO, R_{A}-COO, R_{A}-COO-R_{A}, S, R_{A}-S, R_{A}-S-R_{A}, SO₂, R_{A}-SO₂, R_{A}-SO₂-R_{A}, SO₃H, R_{A}-SO₃H, OSO₃H, R_{A}-OSO₃H, NH, R_{A}-NH, R_{A}-NH-R_{A}, N(R_{A})₂, R_{A}-N(R_{A})₂, NHC(O), R_{A}-NHC(O), R₄-NHC(O)R_{A}, C(O)NH, R_{A}-C(O)NH, R_{A}-C(O)NH-R_{A}, C(O)N(R_{A})₂, R_{A}-C(O)N(R_{A})₂, N(R_{A})₂, R_{A}-N(RA)₂, C(O), R_{A}-C(O), R_{A}-C(O)-R_{A}, wherein R_{A} is selected from the group consisting of: alkyl, alkenyl, alkynyl, cycloalkyl, alkoxyalkyl; and
wherein any of B and R_{A} are independently optionally substituted with one or more groups, preferably 1, 2 or 3 groups, independently selected from COOH, COO(alkyl), SH, S(alkyl), SO₂H, SO₂(alkyl), SO₂(aryl), SO₂(alkaryl), P(OH)(O)₂, halo, halo(alkyl), perhalo(alkyl), OH, O(alkyl), =O, NH₂, =NH, NH(alkyl), N(alkyl)₂, =N(alkyl), NHC(O)(alkyl), C(O)NH₂, C(O)NH(alkyl), C(O)N(alkyl)₂, C(O)NH(aryl), C(O)N(aryl)₂, C(O)NH(aralkyl), C(O)N(aralkyl)₂, C(O)NH(alkaryl), C(O)N(alkaryl)₂, NO₂, CN, SO₂, SO₂NH₂, C(O)H and C(O)(alkyl), alkyl, alkoxyalkyl, alkoxyaryl, alkynyl, aralkyl, alkaryl, heterocyclyl, heteroaryl and aryl.

More preferably, B is selected from the group consisting of: methyl, ethyl, propyl, butyl, hexyl, heptyl, cyclohexyl, cycloheptyl, cyclooctyl, cyclononyl, cyclodecyl, cycloundecyl, tetramethylcyclohexyl, cyclohexylmethyl, hydroxyacetamide, acetamide, methylacetamide, ethylacetamide, propylacetamide, butylacetamide, hexylacetamide, heptylacetamide, amino, 2-amino ethanol, methylamine, ethylamine, propylamine, butylamine, methanol, ethanol, propanol, butanol, heptanol, methylaldehyde, ethylaldehyde, propylaldehyde, butylaldehyde, hydroxylethanal, hydroxypropanal, hydroxylbutanal, phenyl and methyl any of which are optionally substituted. When substituted they may be substituted with one or more groups, preferably 1, 2 or 3 groups, independently selected from =O, COOH, SH, SO₂H, P(OH)(O)₂, halo, trihalomethyl, OH, NH₂, =NH, NH(alkyl), =N(alkyl), NO₂, CN, OCH₃, SO₂NH₂ and C(O)H, alkyl, alkoxyalkyl, alkoxyaryl, aralkyl, alkaryl, heterocyclyl, heteroaryl and aryl.

Advantageously, B is selected from the group consisting of: -O-, -NH-, -C(O)CH₂-, -C(O)-, -C(O)CH₂O-, phenyl and methyl.

Preferably, X and W are independently selected from the group consisting of: alkyl, alkenyl, alkynyl, alkoxyalkyl, cycloalkyl, aryl, heteroaryl, aminoalkyl, heterocyclyl, R_{A}-heteroaryl, R_{A}-heteroaryl-R_{A}, R_{A}-heterocyclyl, R_{A}-heterocyclyl-R_{A}, COO, R_{A}-COO, R_{A}-COO-R_{A}, C(O), R_{A}-C(O), R_{A}-C(O)-R_{A}, NHC(O), R_{A}-NHC(O), R_{A}-NHC(O)-R_{A}, C(O)NH, R_{A}-C(O)NH, R_{A}-C(O)NH-R_{A}, R_{A}-NH, R_{A}-NH-R_{A}, succinimide, R_{A}-succinimide, R_{A}-succinimide-R_{A}, imidazole, R_{A}-imidazole, R_{A}-imidazole-R_{A}, maleimide, R_{A}-maleimide, R_{A}-maleimide-R_{A},
wherein R_{A} is independently selected from the group consisting of: alkyl, alkenyl, alkynyl, alkoxy, alkenyloxy, alkenyloxytolyl, alkenyloxyaryl, and wherein any of the groups are optionally substituted. When substituted they may be substituted with one or more groups, preferably 1, 2 or 3 groups, independently selected from COOH, COO(alkyl), SH, S(alkyl), SO₂H, SO₂(alkyl), SO₂(aryl), SO₂(alkaryl), P(OH)(O)₂, halo, halo(alkyl), perhalo(alkyl), OH, O(alkyl), =O, NH₂, =NH, NH(alkyl), N(alkyl)₂, =N(alkyl), NHC(O)( alkyl), C(O)NH₂, C(O)NH(alkyl), C(O)N(alkyl)₂, C(O)NH(aryl), C(O)N(aryl)₂, C(O)NH(aralkyl), C(O)N(aralkyl)₂, C(O)NH(alkaryl), C(O)N(alkaryl)₂, NO₂ CN, SO₂, SO₂NH₂, C(O)H and C(O)(alkyl), alkyl, alkoxyalkyl, alkoxyaryl, alkynyl, aralkyl, alkaryl, heterocyclyl, heteroaryl and aryl.

More preferably, X and W are independently selected from the group consisting of: methyl, ethyl, propyl, butyl, hexyl, heptyl, methylamine, ethylamine, propylamine, butylamine, hexylamine, heptylamine, ethene, propene, formaldehyde, cyclohexyl, cycloheptyl, phenyl, benzyl, cyclohexyl, cycloheptyl, cyclooctyl, cyclononyl, cyclodecyl, cycloundecyl, tolyl, ethylbenzyl, xylyl, isopropylbenzyl, cyclohexylmethyl, methoxyphenyl, phenethyl, butylphenyl, propylbenzyl, mesitylyl, ethyltolyl, butylbenzyl, diethylbenzyl, dimethylethylbenzyl, tetramethylbenzyl, dipropylbenzyl, triethylbenzyl, furanyl, pyridyl, phthalimido, thienyl, pyrrolyl, imidazolyl, pyrazolyl, thiazolyl, isothiazolyl, oxazolyl, pyrrolidinyl, pyrrolinyl, imidazolidinyl, imidazolinyl, pyrazolidinyl, tetrahydrofuranyl, pyranyl, pyronyl, pyrazinyl, pyridazinyl, piperidinyl, piperazinyl, morpholinyl, thionaphthyl, benzofuranyl, isobenzofuryl, indolyl, oxyindolyl, isoindolyl, indazolyl, indolinyl, azaindolyl, isoindazolyl, benzopyranyl, coumarinyl, isocoumarinyl, quinolyl, isoquinolyl, cinnolinyl, quinazolinyl, pyridopyridyl, benzoxazinyl, quinoxalinyl, chromenyl, chromanyl, isochromanyl, carbolinyl, thiophenyl, thiazolyl, succinimidyl, isoxazolyl, isoxazolonyl, isothiazolyl, oxadiazolyl, thiadiazolyl, pyridazyl, maleimidyl, triazolyl, oxatriazolyl, triazolidinyl, triazolinyl, methoxyltriazolyl, ethoxyltriazolyl, triazolylheptene, triazolyloctene, triazolylnonene, triazolyldecene, methoxyltriazolylheptene, ethoxyltriazolylheptene, methoxyltriazolyloctene, ethoxyltriazolyloctene, methoxyltriazolylnonene, ethoxyltriazolylnonene, methoxyltriazolyldecene, ethoxyltriazolyldecene, methylaminooxomethylsuccinimidethioaminoethanamide, methylaminooxoethylsuccinimidethioaminoethnamide, methylaminooxopropylsuccinimidethioaminoethanamide, methylaminooxomethylsuccinimidethioaminopropanamide, methylaminooxoethylsuecinimidethioaminopropamide, methylaminooxopropylsuccinimidethioaminopropamide, ethylaminooxomethylsuccinimidethioaminoethanamide, ethylaminooxoethylsuccinimidethioaminoethnamide, ethylaminooxopropylsuccinimidethioaminoethanamide, ethylaminooxomethylsuccinimidethioaminopropanamide, ethylaminooxoethylsuccinimidethioaminopropamide; ethylaminooxopropylsuccinimidethioaminopropamide, propylaminooxomethylsuccinimidethioaminoethanamide, propylaminooxoethylsuccinimidethioaminoethnamide, propylaminooxopropylsuccinimidethioaminoethanamide, propylaminooxomethylsuccinimidethioaminopropanamide, propylaminooxoethylsuccinimidethioaminopropamide, propylaminooxopropylsuccinimidethioaminopropamide, methylaminooxomethylmaleimidethioaminoethanamide, methylaminooxoethylmaleimidethioaminoethnamide, methylaminooxopropylmaleimidethioaminoethanamide, methylaminooxomethylmaleimidethioaminopropanamide, methylaminooxoethylmaleimidethioaminopropamide, methylaminooxopropylmaleimidethioaminopropamide, ethylaminooxomethylmaleimidethioaminoethanamide, ethylaminooxoethylmaleimidethioaminoethnamide, ethylaminooxopropylmaleimidethioaminoethanamide, ethylaminooxomethylmaleimidethioaminopropanamide, ethylaminooxoethylmaleimidethioaminopropamide, ethylaminooxopropyl maleimidethioaminopropamide, propylaminooxomethylmaleimidethioaminoethanamide, propylaminooxoethylinaleimidethioaminoethnamide, propylaminooxopropylmaleimidethioaminoethanamide, propylaminooxomethylmaleimidethioaminopropanamide, propylaminooxoethylmaleimidethioaminopropamide, and propylaminooxopropylmaleimidethioaminopropalamide, any of which are optionally substituted. When substituted they may be substituted with one or more groups, preferably 1, 2 or 3 groups, independently selected from =O, COOH, SH, SO₂H, P(OH)(O)₂, halo, trihalomethyl, OH, NH₂, =NH, NH(alkyl), =N(alkyl), NO₂, CN, OCH₃, SO₂NH₂, C(O)H, C(O)CH₃, alkyl, alkoxyalkyl, alkoxyaryl, aralkyl, alkaryl, heterocyclyl, heteroaryl and aryl.

Advantageously, X and W are independently selected from the group consisting of: -CH=CH-CH₂-, -C(O)-, -CH₂-, succinimidyl, -(CH₂)₃NH-, -(CH₂)₂C(O)O-, -CH₂C(O)NH-, -(CH₂)₂C(O)NH-, -(CH₂)₂NHC(O)CH₂CH₂-, -OCH₂-(heteroaryl)-S-(CH₂)₅-C=C-CH₂-, -(CH₂)₂NHC(0)(CH₂)₂-N-succinimide-S-CH₂CH(NH₂)C(O)NH-, -(CH₂)₂NHC(O)(CH₂)₂-N-maleimide-S-CH₂CH(NH₂)C(O)NH-, -(CH₂)₂NHC(O)(CH₂)₂-N-succinimide-S-CH₂CH(NHAc)C(O)NH-, and -(CH₂)₂NH C(O)(CH₂)₂-N-maleimide-S-CH₂CH(NHAc)C(O)NH-, with N-(3-ethylamino-3-oxopropyl)-succinimide-thio-2-amino-propan-3-amido, N-(3-ethylamino-3-oxopropyl)-maleimide-thio-2-amino-propan-3-amido, N-(3-ethylamino-3-oxopropyl)-maleimide-thio-2-amino-propan-3-acetamido, {1-[(6E)oct-6-enyl]-1H-1,2,3-triazol-4-yl}methanol, propylamido, propylamino and ethylamido particularly preferred.

### PROTEIN

The proteins used in the present invention can be any protein molecule, but they preferably possess a particular therapeutic activity. Ideally, the proteins are part of a drug that contains proteins. Drugs that contain proteins are focussed in many therapeutic areas, such as cancer treatment and the treatment of inflammatory and infectious diseases for example, but the therapeutic indication of the drug is determined the protein that it comprises. Preferably, the protein is a recombinant protein.

Although the skilled person will understand that any protein can be used in the present invention, suitable proteins that can be used in the present invention are listed below. It will be understood that this list is not exhaustive and it in no way limits the scope of the present invention.

Therapeutic proteins include: cytokines, growth factors, hormones, chemokines, antibodies, coagulation factors, apoptotic factors and therapeutic peptides. Cytokines include, for example, the interleukines family (*e.g.* IL1, IL-2, IL-4, IL-6 and IL-12), the interferon family (*e.g*. IFN-α, IFN-β and IFN-γ), G-CSF, GM-CSF, TNF-α, IGF-1 and cytokines receptors such as etanercept, a chimeric TNF-α receptor coupled to IgG Fc. Growth factors include, for example, the VEGF family, the FGF family, PDGF, PIGF, and growth factor receptors such as VEGF trap, a chimeric VEGF R1 and R2 receptor coupled to IgG Fc. Hormones include, for example, EPO α and β, hGH, Hepcidin,

Follitropin α and β, Calcitonin, Thyrotropin α (TSH) and hCG. Hormones that are involved in diabetes are also contemplated by the present invention, for example, insulin and glucagon. Chemokines include, for example, SDF-1. Antibodies include, for example, polyclonal, monoclonal (*e.g.* Trastuzumab and Bevacizumab), Fab'2 fragments and Fab fragments such as anti-VEGF Fab. Coagulation factors include, for example, factor VIII, factor VII, factor IX, factor Xa and t-PA. Apoptotic factors include, for example, Fas, Fas-ligands and TRAILs. Therapeutic peptides include, for example, enfuvirtide.

The proteins of the present invention can also be distinguished by their size. Preferably the proteins of the present invention are between 10 and 2000 amino acids long and more preferably the lower limit is selected from: 15, 20, 25, 30, 35, 40, 45, 50, 55, 60, 70, 80, 90, 100, 150 and 200 amino acids; and the upper limit is selected from: 1900, 1800, 1700, 1600, 1500, 1400, 1300, 1200, 1100 and 1000 amino acids.

Drugs that contain proteins that can be used with the present invention include, for example, interferon-α (IFN-α), erythropoietin (EPO) and enfuvirtide. These drugs, like the proteins listed above, have a short half-life in the blood and a high overall clearance rate, which can be problematic resulting in frequent administration that can not be tolerated by the patient.

Preferably, the compound, salt, solvate or prodrug thereof of the present invention is of the following formula: 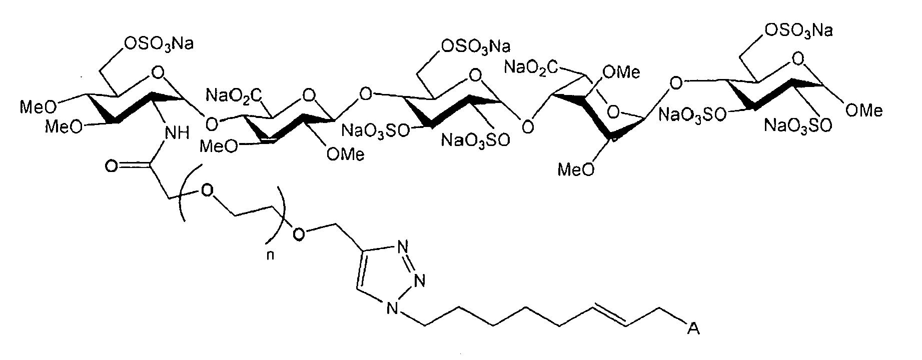 wherein:
A is enfuvirtide; and
n is 6; or 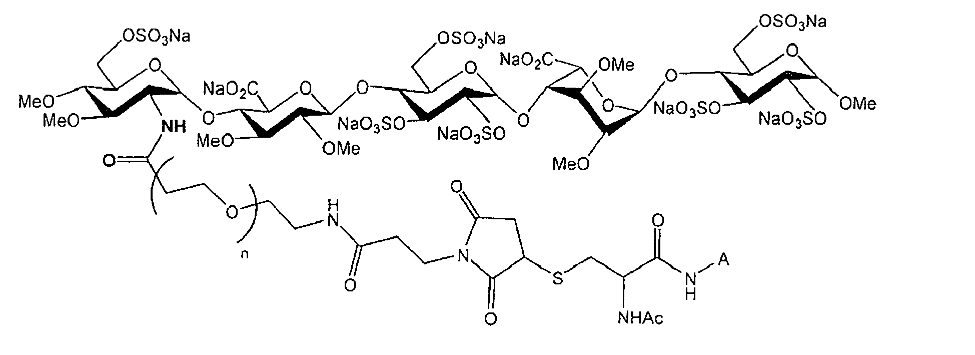
wherein:
A is enfuvirtide; and
n is 12; or 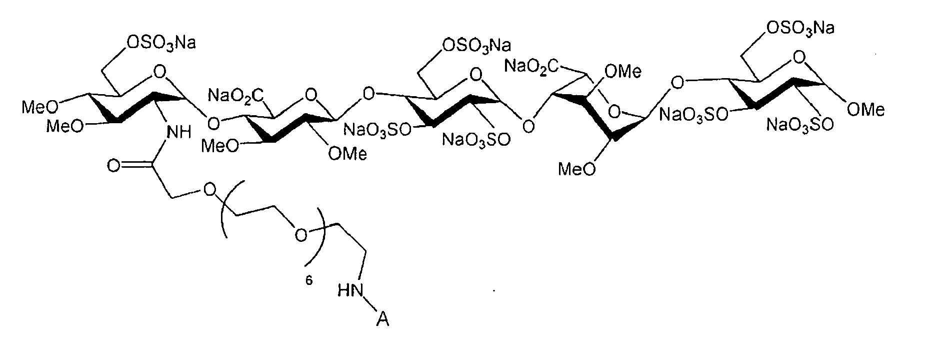
wherein:
A is interferon-α; and
n is 6; or 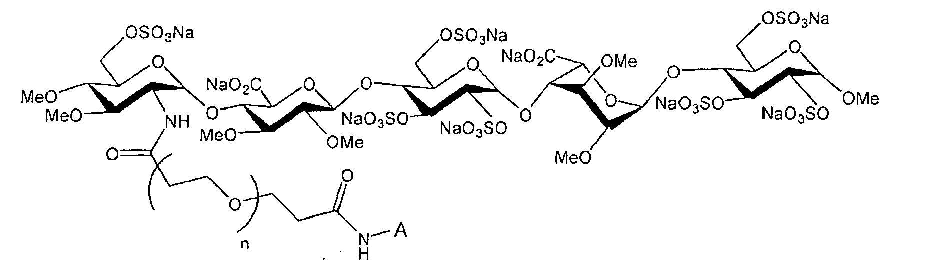
wherein:
A is erythropoietin; and
n is 5.

The present invention further provides a pharmaceutical composition comprising a compound, salt, solvate or prodrug, as described above, and a pharmaceutically acceptable diluent or carrier. Additionally, the present invention provides a method of making the aforementioned pharmaceutical composition, comprising mixing said compound, salt, solvate or pro-drug with a pharmaceutically acceptable diluent or carrier.

In an alternative aspect of the present invention, the use of an AT-binding oligosaccharide conjugated to a protein, *via* a linker, to increase the biological half-life of the protein is provided. Additionally, a method of increasing the biological half-life of a protein is provided by conjugating an AT-binding oligosaccharide to a protein, *via* a linker. The AT-binding oligosaccharide, the linker and the protein are all described above.

The compounds of the present invention are suitable for use in therapy or in a method of treating a disease. It will be apparent to the skilled person that the disease or therapeutic indication that the compounds of the present invention can be used to treat, or used in the manufacture of a medicament for, is determined by the protein that is conjugated to the AT-binding oligosaccharide *via* the linker.

### DEFINITIONS

The present invention also provides the use of a compound comprising an AT-binding oligosaccharide conjugated to a protein, *via* a linker, a salt, solvate or pro-drug thereof, substantially as described herein before, for increasing the biological half-life of the protein.

### Pharmaceutical compositions.

The compounds of the present invention may also be present in the form of pharmaceutically acceptable salts. For use in medicine, the salts of the compounds of this invention refer to non-toxic "pharmaceutically acceptable salts." FDA approved pharmaceutically acceptable salt forms (Gould, P. L. International J. Pharm., 1986, 33, 201-217; Berge, S.M. et al. J. Pharm. Sci., 1977, 66(1), 1-19) include pharmaceutically acceptable acidic/anionic or basic/cationic salts.

Pharmaceutically acceptable salts of the acidic or basic compounds of the invention can of course be made by conventional procedures, such as by reacting the free base or acid with at least a stoicheometric amount of the desired salt-forming acid or base.

Pharmaceutically acceptable salts of the acidic compounds of the invention include salts with inorganic cations such as sodium, potassium, calcium, magnesium, zinc, and ammonium, and salts with organic bases. Suitable organic bases include N-methyl-D-glucamine, arginine, benzathine, diolamine, olamine, procaine and tromethamine.

Pharmaceutically acceptable salts of the basic compounds of the invention include salts derived from organic or inorganic acids. Suitable anions include acetate, adipate, besylate, bromide, camsylate, chloride, citrate, edisylate, estolate, fumarate, gluceptate, gluconate, glucuronate, hippurate, hyclate, hydrobromide, hydrochloride, iodide, isethionate, lactate, lactobionate, maleate, mesylate, methylbromide, methylsulfate, napsylate, nitrate, oleate, pamoate, phosphate, polygalacturonate, stearate, succinate, sulfate, sulfosalicylate, tannate, tartrate, terephthalate, tosylate and triethiodide. Hydrochloride salts are particularly preferred.

The invention also comprehends derivative compounds ("pro-drugs") which are degraded *in vivo* to yield the species of Formula (I). Pro-drugs are usually (but not always) of lower potency at the target receptor than the species to which they are degraded. Pro-drugs are particularly useful when the desired species has chemical or physical properties, which make its administration difficult or inefficient. For example, the desired species may be only poorly soluble, it may be poorly transported across the mucosal epithelium, or it may have an undesirably short plasma half-life. Further discussion of pro-drugs may be found in Stella, V. J. et al. "Prodrugs", Drug Delivery Systems, 1985, 112-176, Drugs, 1985, 29, 455-473 and "Design of Prodrugs", ed. H. Bundgaard, Elsevier, 1985.

Pro-drug forms of the pharmacologically active compounds of the invention will generally be compounds according akin to those described in the claims.

Compounds of Formula (I) having an amino group may be derivatised with a ketone or an aldehyde such as formaldehyde to form a Mannich base. This will hydrolyse with first order kinetics in aqueous solution.

Thus, in the methods of treatment of the present invention, the term "administering'' shall encompass the treatment of the various disorders described with the compound specifically disclosed or with a compound which may not be specifically disclosed, but which converts to the specified *compound in vivo* after administration to the subject.

Pharmaceutically acceptable ester derivatives in which one or more free hydroxy groups are esterified in the form of a pharmaceutically acceptable ester are particularly pro-drug esters that may be convertible by solvolysis under physiological conditions to the compounds of the present invention having free hydroxy groups.

It is anticipated that the compounds of the invention can be administered by oral or parenteral routes, including intravenous, intramuscular, intraperitoneal, subcutaneous, rectal and topical administration, and inhalation.

For oral administration, the compounds of the invention will generally be provided in the form of tablets or capsules or as an aqueous solution or suspension.

Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate and lactose. Corn starch and alginic acid are suitable disintegrating agents. Binding agents may include starch and gelatine. The lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatine capsules in which the active ingredient is mixed with a solid diluent and soft gelatine capsules wherein the active ingredient is mixed with water or an oil such as peanut oil, liquid paraffin or olive oil.

For intramuscular, intraperitoneal, subcutaneous and intravenous use, the compounds of the invention will generally be provided in sterile aqueous solutions or suspensions, buffered to an appropriate pH and isotonicity. Suitable aqueous vehicles include Ringer's solution and isotonic sodium chloride. Aqueous suspensions according to the invention may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

The pharmaceutical compositions of the present invention may, in particular, comprise more than one agent (multiple) of the present invention, e.g., two or more agents. The invention also provides a pharmaceutical preparation or system, comprising (a) a first agent, which is an agent of the invention; and (b) a second pharmaceutical agent. Said multiple agents of the invention or said first and second agents are formulated either in admixture or as separate compositions, *e.g.* for simultaneous though separate, or for sequential administration (see below).

### Modes of Administration

The compositions of the present invention can be delivered directly or in pharmaceutical compositions containing excipients (see above), as is well known in the art. The present methods of treatment involve administration of a therapeutically effective amount of an agent of the present invention to a subject.

The term "therapeutically effective amount" as used herein refers to an amount of an agent according to the present invention needed to treat, ameliorate, or prevent the targeted disease condition, or to exhibit a detectable therapeutic or preventative effect. In general, the therapeutically effective dose can be estimated initially either in cell culture assays or in animal models, for example, in non-human primates, mice, rabbits, dogs, or pigs. The animal model may also be used to determine the appropriate concentration range and route of administration. Such information can then be used to determine useful doses and routes for administration in humans.

Effective doses of the compounds of the present invention may be ascertained by conventional methods. The specific dosage level required for any particular patient will depend on a number of factors, including severity of the condition being treated, the route of administration, the general health of the patient (i.e. age, weight and diet), the gender of the patient, the time and frequency of administration, and tolerance/response to therapy. In general, however, the daily dose (whether administered as a single dose or as divided doses) will be in the range 0.001 to 5000 mg per day, more usually from 1 to 1000 mg per day, and most usually from 10 to 200 mg per day. Alternatively, dosages can be administered per unit body weight and, in this instance, a typical dose will be between 0.01 µg/kg and 50 mg/kg, especially between 10 µg/kg and 10 mg/kg, between 100 µg/kg and 2 mg/kg.

An advantage of the compounds of the present invention is that they permit administration to be limited to one, two, three or four times weekly or monthly.

An effective and convenient route of administration and an appropriate formulation of the agents of the invention in pharmaceutical compositions (see above) may also be readily determined by routine experimentation. Various formulations and drug delivery systems are available in the art (see, *e.g.,* Gennaro AR (ed.). Remington: The Science and Practice of Pharmacy. Lippincott Williams & Wilkins. 21 st edition. July 3, 2005 and Hardman JG, Limbird LE, Alfred G. Gilman AG. Goodman & Gilman's The Pharmacological Basis of Therapeutics. McGraw-Hill; 10th edition. August 13,2001).

Suitable routes of administration may, for example, include vaginal, rectal, intestinal, oral, nasal (intranasal), pulmonary or other mucosal, topical, transdermal, ocular, aural, and parenteral administration.

Primary routes for parenteral administration include intravenous, intramuscular, and subcutaneous administration. Secondary routes of administration include intraperitoneal, intra-arterial, intra-articular, intracardiac, intracisternal, intradermal, intralesional, intraocular, intrapleural, intrathecal, intrauterine, and intraventricular administration. The indication to be treated, along with the physical, chemical, and biological properties of the drug, dictate the type of formulation and the route of administration to be used, as well as whether local or systemic delivery would be preferred.

For compositions useful for the present methods of treatment, a therapeutically effective dose can be estimated initially using a variety of techniques well known in the art. Initial doses used in animal studies may be based on effective concentrations established in cell culture assays. Dosage ranges appropriate for human patients can be determined, for example, using data obtained from animal studies and cell culture assays.

A therapeutically effective dose or amount of an agent, agent, or drug of the present invention refers to an amount or dose of the agent, agent, or drug that results in amelioration of symptoms or a prolongation of survival in a patient. Toxicity and therapeutic efficacy of such molecules can be determined by standard pharmaceutical procedures in cell cultures or experimental animals, *e.g.,* by determining the LD50 (the dose lethal to 50% of the population) and the ED50 (the dose therapeutically effective in 50% of the population). The dose ratio of toxic to therapeutic effects is the therapeutic index, which can be expressed as the ratio LD50/ED50. Agents that exhibit high therapeutic indices are preferred.

The effective amount or therapeutically effective amount is the amount of the agent or pharmaceutical composition that will elicit the biological or medical response of a tissue, system, animal, or human that is being sought by the researcher, veterinarian, medical doctor, or other clinician, *e.g*., regulation of glucose metabolism, decrease in elevated or increased blood glucose levels, treatment or prevention of a disorder associated with altered glucose metabolism, *e.g.*, diabetes, etc.

Dosages preferably fall within a range of circulating concentrations that includes the ED50 with little or no toxicity. Dosages may vary within this range depending upon the dosage form employed and/or the route of administration utilised. The exact formulation, route of administration, dosage, and dosage interval should be chosen according to methods known in the art, in view of the specifics of a patient's condition.

Dosage amount and interval may be adjusted individually to provide plasma levels of the active moiety that are sufficient to achieve the desired effects, i.e., minimal effective concentration (MEC). The MEC will vary for each agent but can be estimated from, for example, in vitro data and animal experiments. Dosages necessary to achieve the MEC will depend on individual characteristics and route of administration. In cases of local administration or selective uptake, the effective local concentration of the drug may not be related to plasma concentration.

The amount of agent or composition administered may be dependent on a variety of factors, including the sex, age, and weight of the patient being treated, the severity of the affliction, the manner of administration, and the judgement of the prescribing physician.

The present compositions may, if desired, be presented in a pack or dispenser device containing one or more unit dosage forms containing the active ingredient. Such a pack or device may, for example, comprise metal or plastic foil, such as a blister pack, or glass and rubber stoppers such as in vials. The pack or dispenser device may be accompanied by instructions for administration. Compositions comprising an agent of the invention formulated in a compatible pharmaceutical carrier may also be prepared, placed in an appropriate container, and labelled for treatment of an indicated condition.

### Chemical Definitions

Formulaic representation of apparent orientation of a functional group is not necessarily intended to represent actual orientation. Thus, for example, a divalent amide group represented as C(O)NH is also intended to cover NHC(O).

In the interests of simplicity, terms which are normally used to refer to monovalent groups (such as "alkyl" or "alkenyl") are also used herein to refer to divalent, trivalent or tetravalent bridging groups which are formed from the corresponding monovalent group by the loss of one or more hydrogen atom(s). Whether such a term refers to a monovalent group or to a polyvalent group will be clear from the context. Where a polyvalent bridging group is formed from a cyclic moiety, the linking bonds may be on any suitable ring atom, patient to the normal rules of valency.

Where any particular moiety is substituted, for example an imidazole group comprising a substituent on the heteroaryl ring, unless specified otherwise, the term "substituted" contemplates all possible isomeric forms. For example, substituted imidazole includes all of the following permutations:

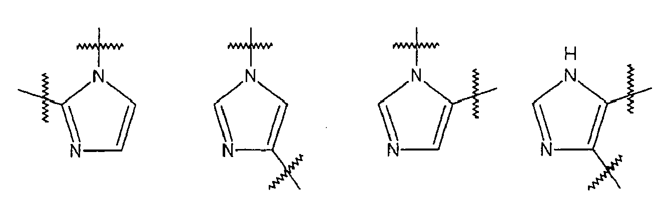

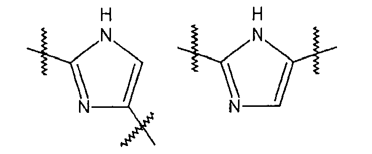

The terms "comprising" and "comprises" means "including" as well as "consisting" *e.g.* a composition "comprising" X may consist exclusively of X or may include something additional *e.g.* X + Y.

The word "substantially" does not exclude "completely" *e.g.* a composition which is "substantially free" from Y may be completely free from Y. Where necessary, the word "substantially" may be omitted from the definition of the invention.

"Optional" or "optionally" means that the subsequently described event of circumstances may or may not occur, and that the description includes instances where said event or circumstance occurs and instances in which it does not.

"May" means that the subsequently described event of circumstances may or may not occur, and that the description includes instances where said event or circumstance occurs and instances in which it does not.

Where reference is made to a group in a chain being "not present" the group is replaced a bond between the two adjoining groups.

Where the compounds according to this invention have at least one chiral centre, they may accordingly exist as enantiomers. Where the compounds possess two or more chiral centres, they may additionally exist as diastereomers. Where the processes for the preparation of the compounds according to the invention give rise to mixture of stereoisomers, these isomers may be separated by conventional techniques such as preparative chromatography. The compounds may be prepared in racemic form or individual enantiomers may be prepared by standard techniques known to those skilled in the art, for example, by enantiospecific synthesis or resolution, formation of diastereomeric pairs by salt formation with an optically active acid, followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column. It is to be understood that all such isomers and mixtures thereof are encompassed within the scope of the present invention.

As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. The permissible substituents can be one or more and the same or different for appropriate organic compounds. For purposes of this invention, heteroatoms such as nitrogen may have hydrogen substituents and/or any permissible substituents of organic compounds described herein which satisfy the valencies of the heteroatoms. This invention is not intended to be limited in any manner by the permissible substituents of organic compounds.

As used herein, when referring to a substitution, it means that the hydrocarbon chain is interrupted by one or more of the groups indicated. Where more than one substitution occurs, it may be adjacent to another or remote, i.e., separated by 0, 1, 2, 3, 4, 5, 6, 7, 8, 9, 10 or more carbon atoms.

Furthermore, the term "substituted" comprehends a substitution that may be adjacent or remote to the point of attachment of the group being substituted to the rest of the molecule. It also comprehends the group being the point of attachment to the rest of the molecule.

Where a group comprises two or more moieties defined by a single carbon atom number, for example, C₂₋₂₀-alkoxyalkyl, the carbon atom number indicates the total number of carbon atoms in the group.

As used herein, the term "heteroatom" includes N, O, S, P, Si and halogen (including F, Cl, Br and I). In the context of a hydrocarbon chain interrupted by one or more heteroatoms, the term "heteroatoms" includes N, O and S.

The term "halogen" or "halo" is used herein to refer to any of fluorine, chlorine, bromine and iodine. Most usually, however, halogen substituents in the compounds of the invention are chlorine, bromine and fluorine substituents. Groups such as halo(alkyl) includes mono-, di- or tri-halo substituted alkyl groups. Moreover, the halo substitution may be at any position in the alkyl chain. "Perhalo" means completely halogenated, e.g., trihalomethyl and pentachloroethyl.

As used herein, the term "alkyl" refers to a cyclic, straight or branched saturated monovalent hydrocarbon radical, having the number of carbon atoms as indicated. For example, the term "C₁₋₂₀-alkyl" includes C₁, C₂, C₃, C₄, C₄, C₅, C₆, C₇, C₈, C₉, C₁₀, C₁₁, C₁₂, C₁₃, C₁₄, C₁₅, C₁₆, C_{17,} C₁₈, C₁₉ and C₂₀ alkyl groups. By way of non-limiting example, suitable alkyl groups include methyl, ethyl, propyl, *iso*-propyl, butyl, *iso*-butyl, *tert*-butyl, pentyl, hexyl, octyl, nonyl, dodecyl, eicosyl, cyclopropyl, cyclobutyl, cyclopentyl, cyclohexyl, methylcyclohexyl, dimethylcyclohexyl, trimethylcyclohexyl, cycloheptyl, cyclooctyl, cyclononyl, cyclododecyl, spiroundecyl, bicyclooctyl and adamantyl, cyclopropylmethyl, cyclopropylethyl, cyclobutylmethyl, cyclobutylethyl, cyclopentylmethyl, cyclopentylethyl, cyclohexylmethyl, cyclohexylethyl, cyclohexylpropyl, cyclohexylbutyl, methylcyclohexylmethyl, dimethylcyclohexylmethyl, trimethylcyclohexylmethyl, cycloheptylmethyl, cycloheptylethyl, cycloheptylpropyl, cycloheptylbutyl and adamantylmethyl. Preferred ranges of alkyl groups of the present invention are: C₁₋₂₀-alkyl, C₁₋₁₈-alkyl, C₁₋₁₅-alkyl, C₁₋₁₂-alkyl, C₁₋₉-alkyl, C₁₋₈-alkyl, C₁₋₅-alkyl, C₁₋₄-alkyl, C₁₋₃-alkyl and C₁₋₂-alkyl. Preferred ranges in cycloalkyl groups are: C₃₋₂₀, C₃₋₁₀, C₄₋₇ and C₅₋₆.

As used herein, the term "alkenyl" refers to a cyclic, straight or branched unsaturated monovalent hydrocarbon radical, having the number of carbon atoms as indicated, and the distinguishing feature of a carbon-carbon double bond. For example, the term "C₂₋₂₀-alkenyl" includes C₂, C₃, C₄, C₄, C₅, C₆, C₇, C₈, C₉, C₁₀, C₁₁, C₁₂, C₁₃, C₁₄, C₁₅, C₁₆, C₁₇, C₁₈, C₁₉ and C₂₀ alkenyl groups. By way of non-limiting example, suitable alkenyl groups include ethenyl, propenyl, butenyl, penentyl, hexenyl, octenyl, nonenyl, dodecenyl and eicosenyl, wherein the double bond may be located anywhere in the carbon chain. Preferred ranges of alkenyl groups of the present invention are: C₁₋₂₀-alkenyl, C₁₋₁₈-alkenyl, C₁₋₁₅-alkenyl, C₁₋₁₂-alkenyl, C₁₋₉-alkenyl, C₁₋₈-alkenyl, C₁₋₅-alkenyl, C₁₋₄-alkenyl, C₁₋₃-alkenyl and C₁₋₂-alkenyl.

As used herein, the term "alkynyl" refers to a straight or branched unsaturated monovalent hydrocarbon radical, having the number of carbon atoms as indicated, and the distinguishing feature of a carbon-carbon triple bond. For example, the term "C₂₋₂₀ alkynyl" includes C₂, C₃, C₄, C₄, C₅, C₆, C₇, C₈, C₉, C₁₀, C₁₁, C₁₂, C₁₃, C₁₄, C₁₅, C₁₆, C₁₇, C₁₈, C₁₉ and C₂₀ alkynyl groups. By way of non-limiting example, suitable alkynyl groups include ethynyl, propynyl, butynyl, penynyl, hexynyl, octynyl, nonynyl, dodycenyl and eicosynyl, wherein the triple bond may be located anywhere in the carbon chain. Preferred ranges of alkynyl groups of the present invention are: C₁₋₂₀-alkynyl, C₁₋₁₈-alkynyl, C₁₋₁₅-alkynyl, C₁₋₁₂-alkynyl, C₁₋₉-alkynyl, C₁₋₈-alkynyl, C₁₋₅-alkynyl, C₁₋₄-alkynyl, C₁₋₃-alkynyl and C₁₋₂-alkynyl.

Alkoxy refers to the group "alkyl-O-", where alkyl is as defined above. By way of non-limiting example, suitable alkoxy groups include methoxy, ethoxy, propoxy, butoxy, pentoxy and hexoxy.

As used herein, the term "alkoxyalkyl" refers to an alkyl group having an alkoxy substituent. Binding is through the alkyl group. The alkyl moiety may be cyclic, straight or branched. The alk and alkyl moieties of such a group may be substituted as defined above, with regard to the definition of alkyl. By way of non-limiting example, suitable alkoxyalkyl groups include methoxymethyl, methoxyethyl, ethoxymethyl, ethoxyethyl, methoxypropyl and ethoxypropyl.

As used herein, the term "alkoxyaryl" refers to an aryl group having an alkoxy substituent. Binding is through the aryl group. The alkoxy and aryl moieties of such a group may be substituted as defined herein, with regard to the definitions of alkoxy and aryl. The alkyl moiety may be cyclic, straight or branched. By way of non-limiting example, suitable alkoxyaryl groups include methoxyphenyl, ethoxyphenyl, dimethoxyphenyl and trimethoxyphenyl.

As used herein, the term "aryl" refers to monovalent unsaturated aromatic carbocyclic radical having one, two, three, four, five or six rings, preferably one, two or three rings, which may be fused or bicyclic. Preferably, the term "aryl" refers to an aromatic monocyclic ring containing 6 carbon atoms, which may be substituted on the ring with 1, 2, 3, 4 or 5 substituents as defined herein; an aromatic bicyclic or fused ring system containing 7, 8, 9 or 10 carbon atoms, which may be substituted on the ring with 1, 2, 3, 4, 5, 6, 7, 8 or 9 substituents as defined herein; or an aromatic tricyclic ring system containing 10, 11, 12, 13 or 14 carbon atoms, which may be substituted on the ring with 1, 2, 3, 4, 5, 6, 7, 8, 9, 10, 11, 12 or 13 substituents as defined herein. By way of non-limiting example, suitable aryl groups include phenyl, biphenyl, binaphthyl, indanyl, phenanthryl, fluoryl, flourenyl, stilbyl, benzylphenanthryl, acenaphthyl, azulenyl, phenylnaphthyl, benzylfluoryl, tetrahydronaphthyl, perylenyl, picenyl, chrysyl, pyrenyl, tolyl, chlorophenyl, dichlorophenyl, trichlorophenyl, methoxyphenyl, dimethoxyphenyl, trimethoxyphenyl, fluorophenyl, difluorophenyl, trifluorophenyl, nitrophenyl, dinitrophenyl, trinitrophenyl, aminophenyl, diaminophenyl, triaminophenyl, cyanophenyl, chloromethylphenyl, tolylphenyl, xylylphenyl, chloroethylphenyl, trichloromethylphenyl, dihydroindenyl, benzocycloheptyl and trifluoromethylphenyl. Preferred ranges of aryl groups of the present invention are: C₆₋₂₅-aryl, C₆₋₂₃-aryl, C₆₋₂₀-aryl, C₆₋₁₈-aryl, C₆₋₁₅-aryl, C₆₋₁₂-aryl, C₆₋₁₀-aryl, C₆₋₉-aryl, C₆₋₈-aryl and C₆₋₇-aryl.

The term "heteroaryl" refers to a monovalent unsaturated aromatic heterocyclic radical having one, two, three, four, five or six rings, preferably one, two or three rings, which may be fused or bicyclic. Preferably, "heteroaryl" refers to an aromatic monocyclic ring system containing five members of which at least one member is a N, O or S atom and which optionally contains one, two or three additional N atoms, an aromatic monocyclic ring having six members of which one, two or three members are a N atom, an aromatic bicyclic or fused ring having nine members of which at least one member is a N, O or S atom and which optionally contains one, two or three additional N atoms or an aromatic bicyclic ring having ten members of which one, two or three members are a N atom. By way of non-limiting example, suitable heteroaryl groups include furanyl, pryingly, pyridyl, phthalimido, thiophenyl, pyrrolyl, imidazolyl, pyrazolyl, thiazolyl, isothiazolyl, oxazolyl, oxadiazolyl, pyronyl, pyrazinyl, tetrazolyl, thionaphthyl, benzofuranyl, isobenzofuryl, indolyl, oxyindolyl, isoindolyl, indazolyl, indolinyl, azaindolyl, isoindazolyl, benzopyranyl, coumarinyl, isocoumarinyl, quinolyl, isoquinolyl, cinnolinyl, quinazolinyl, pyridopyridyl, benzoxazinyl, quinoxadinyl, chromenyl, chromanyl, isochromanyl, carbolinyl, thiazolyl, isoxazolyl, isoxazolonyl, isothiazolyl, triazolyl, oxadiazolyl, thiadiazolyl, benzodioxepinyl and pyridazyl. Preferred ranges of heteroaryl groups of the present invention are: C₆₋₃₀-heteroaryl, C₆₋₂₅-heteroaryl, C₆₋₂₀-heteroaryl, C₆₋₁₈-heteroaryl, C₆₋₁₅-heteroaryl, C₆₋₁₂-heteroaryl, C₆₋₁₀-heteroaryl, C₆₋₉-heteroaryl, C₆₋₈-heteroaryl and C₆₋₇-heteroaryl.

The term "heterocyclyl" refers to a saturated or partially unsaturated ring having three members of which at least one member is a N, O or S atom and which optionally contains one additional O atom or additional N atom; a saturated or partially unsaturated ring having four members of which at least one member is a N, O or S atom and which optionally contains one additional O atom or one or two additional N atoms; a saturated or partially unsaturated ring having five members of which at least one member is a N, O or S atom and which optionally contains one additional O atom or one, two or three additional N atoms; a saturated or partially unsaturated ring having six members of which one, two or three members are an N, O or S atom and which optionally contains one additional O atom or one, two or three additional N atoms; a saturated or partially unsaturated ring having seven members of which one, two or three members are an N, O or S atom and which optionally contains one additional O atom or one, two or three additional N atoms; a saturated or partially unsaturated ring having eight members of which one, two or three members are an N, O or S atom and which optionally contains one additional O atom or one, two or three additional N atoms; a saturated or partially unsaturated bicyclic ring having nine members of which at least one member is a N, O or S atom and which optionally contains one, two or three additional N atoms; or a saturated or partially unsaturated bicyclic ring having ten members of which one, two or three members are an N, O or S atom and which optionally contains one additional O atom or one, two or three additional N atoms. Preferably, heterocycles comprising peroxide groups are excluded from the definition of heterocyclyl. By way of non-limiting example, suitable heterocyclyl groups include pyrrolinyl, pyrrolidinyl, dioxolanyl, tetrahydrofuranyl, morpholinyl, imidazolinyl, imidazolidinyl, maleimidyl, pyrazolidinyl, piperidinyl, dihydropyranyl, succinimidyl, tetrahydropyranyl, thiopyranyl, tetrahydrothiopyranyl and piperazinyl. Preferred ranges of heterocyclyl groups of the present invention are: C₆₋₃₀-heterocyclyl, C₆₋₂₅-heterocyclyl, C₆₋₂₀-heterocyclyl, C₆₋₁₈-heterocyclyl, C₆₋₁₅-heterocyclyl, C₆₋₁₂-heterocyclyl, C₆₋₁₀-heterocyclyl, C₆₋₉-heterocyclyl, C₆₋₈-heterocyclyl and C₆₋₇-heterocyclyl.

As used herein, the term "alkaryl" and "alkylaryl" refer to an aryl group with an alkyl substituent. Binding is through the aryl group. Such groups have the number of carbon atoms as indicated. The alkyl and aryl moieties of such a group may be substituted as defined herein, with regard to the definitions of alkyl and aryl. The alkyl moiety may be straight or branched. Particularly preferred examples of alkaryl include tolyl, xylyl, butylphenyl, mesityl, ethyltolyl, methylindanyl, methylnaphthyl, methyltetrahydronaphthyl, ethylnaphthyl, dimethylnaphthyl, propylnaphthyl, butylnaphthyl, methylfluoryl and methylchrysyl. Again, preferred ranges carbon atoms for alkaryl and alkylaryl groups of the present invention are: C₆₋₃₀, C₆₋₂₅, C₆₋₂₀, C₆₋₁₈, C₆₋₁₅, C₆₋₁₂, C₆₋₁₀, C₆₋₉, C₆₋₈ and C₆₋₇.

As used herein, the term "aralkyl" and "arylalkyl" refer to an alkyl group with an aryl substituent. Binding is through the alkyl group. Such groups have the number of carbon atoms as indicated. The aryl and alkyl moieties of such a group may be substituted as defined herein, with regard to the definitions of aryl and alkyl. The alkyl moiety may be straight or branched. Particularly preferred examples of aralkyl include benzyl, methylbenzyl, ethylbenzyl, dimethylbenzyl, diethylbenzyl, methylethylbenzyl, methoxybenzyl, chlorobenzyl, dichlorobenzyl, trichlorobenzyl, phenethyl, phenylpropyl, diphenylpropyl, phenylbutyl, biphenylmethyl, fluorobenzyl, difluorobenzyl, trifluorobenzyl, phenyltolylmethyl, trifluoromethylbenzyl, bis(trifluoromethyl)benzyl, propylbenzyl, tolylmethyl, fluorophenethyl, fluorenylmethyl, methoxyphenethyl, dimethoxybenzyl, dichlorophenethyl, phenylethylbenzyl, isopropylbenzyl, diphenylmethyl, propylbenzyl, butylbenzyl, dimethylethylbenzyl, phenylpentyl, tetramethylbenzyl, phenylhexyl, dipropylbenzyl, triethylbenzyl, cyclohexylbenzyl, naphthylmethyl, diphenylethyl, triphenylmethyl and hexamethylbenzyl. Similarly, preferred ranges carbon atoms for aralkyl and arylalkyl groups of the present invention are: C₆₋₃₀, C₆₋₂₅, C₆₋₂₀, C₆₋₁₈, C₆₋₁₅, C₆₋₁₂, C₆₋₁₀, C₆₋₉, C₆₋₈ and C₆₋₇.

The term "aminoalkyl" refers to an alkyl group with an amine substituent. Binding is through the alkyl group. Such groups have the number of carbon atoms as indicated above for "alkyl" groups. The alkyl moiety of such a group may be substituted as defined herein, with regard to the definition of alkyl. By way of non-limiting example, suitable aminoalkyl groups include aminomethyl, aminoethyl, aminopropyl, aminobutyl, aminopentyl and aminohexyl.

The term "aminoaryl" refers to an amine group with an aryl substituent. Binding is through the alkyl group. Such groups have the number of carbon atoms as indicated above for "aryl" groups. The aryl moiety of such a group may be substituted as defined herein, with regard to the definition of aryl.

With regard to one or more substituents which are referred to as being on the carbon backbone of a group with a compound definition, for example, "alkaryl", the substituent may be on either or both of the component moieties, *e.g.,* on the alkyl and/or aryl moieties.

Reference to cyclic systems, *e.g.,* aryl, heteroaryl, *etc.,* contemplates monocyclic and polycyclic systems. Such systems comprise fused, non-fused and spiro conformations, such as bicyclooctyl, adamantyl, biphenyl and benzofuran.

The term "monosaccharide" means a sugar molecule having a chain of 3-10 carbon atoms in the form of an aldehyde (aldose) or ketone (ketose). Suitable monosaccharides for use in the invention include both naturally occurring and synthetic monosaccharides. Such monosaccharides include trioses, such as glycerose and di hydroxyacetone; textroses, such as erythanose and erythrulose; pentoses, such as xylose, arabinose, ribose, xylulose ribulose; methyl pentoses (6-deoxyhexoses), such as rhamnose and fructose; hexoses, such as glucose, mannose, galactose, fructose and sorbose; heptoses, such as glucoheptose, galamannoheptose, sedoheptulose and mannoheptulose. Preferred monosaccharides are hexoses.

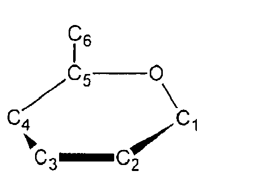

The monosaccharides may be attached to another monosaccharide group at the C₁, C₂, C₃, C₄, C₅ and C₆ position (shown above) to form a glycosyl bond and an oligosaccharide. Typically, a monosaccharide is attached to the C₃, C₄, C₅ and C₆ position through an oxygen atom attached to the C₁ carbon of another monosaccharide, which forms a glycosidic linkage and an oligosaccharide. Oligosaccharides that can be used in the present invention include disaccharides, trisaccharides, tetrasaccharides, pentasaccharides, hexasaccharides, heptasaccharides and octasaccharides. Preferred oligosaccharides are pentasaccharides.

As used herein, the term "amino acid" encompasses a naturally occurring or a non-naturally occurring amino acid, such as amino adipic acid for example. Additionally, the amino acid may be synthetic. The amino acids of the present invention may be L-amino acids or D-amino acids. Preferably, the amino acid is selected from the group consisting of: alanine, arginine, asparagine, aspartic acid, cysteine, cystine, glycine, glutamic acid, glutamine, histidine, isoleucine, leucine, lysine, methionine, phenylalanine, proline, serine, threonine, tryptophan, tyrosine, valine.

As used herein, the term "protein" encompasses "protein", "peptide" and "polypeptide" and refers to amino acid polymers of any length. The polymer may be linear or branched, it may comprise modified amino acids and it may be interrupted by non-amino acids. This term also encompasses an amino acid polymer that has been modified naturally or by intervention by, for example, disulfide bond formation, glycosylation, lipidation, acetylation, phosphorylation, or any other manipulation or modification, such as conjugation with a labelling component. Also included within the definition are, for example, polypeptides containing one or more analogues of an amino acid (including, for example, unnatural amino acids, etc.), as well as other modifications known in the art. Proteins of the invention can be naturally or non-naturally glycosylated (i.e. the polypeptide has a glycosylation pattern that differs from the glycosylation pattern found in the corresponding naturally occurring polypeptide).

As used herein, the terms "poly-amino acid", "amino acid chain" and "chain of amino acids" refer to a linear or branched amino acid polymer of any length. Preferably, the polymer comprises from 2 to 40 amino acids, from 3 to 10 amino acids, from 4 to 8 amino acids and more preferably from 5 to 7 amino acids. This term encompasses an amino acid polymer that has been modified naturally or by intervention by, for example, disulfide bond formation, glycosylation, lipidation, acetylation, phosphorylation, or any other manipulation or modification, such as conjugation with a labelling component. The amino acid polymer may be a homopolymer (*i.e*. made up of a monomeric unit comprising a single amino acid) or it may be a heteropolymer (*i.e*. made up of a monomeric unit comprising more than one amino acid, wherein at least two of amino acids in the monomeric unit are structurally different).

It will be appreciated that the terms: "interferon family", "interferon" and "interferons" encompass all interferons in this class, such as Type-I and Type-II interferons. It will be apparent to the skilled person what interferons are encompassed, but by way of non-limiting example, suitable interferons include: IFN-α (*e.g*. IFN-α(1, 2, 4, 5, 6, 7, 8, 10, 13, 14, 16, 17 and 21)), IFN-β, IFN-ω, IFN-ε, IFN-κ, IFN-ζ, IFN-ν, IFN-τ, IFN-δ, IFN-γ and IFN-λ. More specifically, the term "interferon-α" (IFN-α) includes, for example, all forms of IFN-α, such as: IFN-α(1, 2, 4, 5, 6, 7, 8, 10, 13, 14, 16, 17 and 21).

As used herein, the term "capable of binding to antithrombin (AT)" means compounds that can bind to AT. It has been found that a pentasaccharide is the optimal structural element that can be used for binding to the binding domain of AT.

It will be appreciated that ionisable groups may exist in the neutral form shown in formulae herein, or may exist in charged form *e.g.* depending on pH. Thus a carboxylate group may be shown as -COOH, this formula is merely representative of the neutral carboxylate group, and other charged forms are encompassed by the invention (*i*.*e*. COO⁻).

Similarly, references herein to cationic and anionic groups should be taken to refer to the charge that is present on that group under physiological conditions *e.g.* where a sulphate group -OSO₃H is deprotonated to give the anionic -OSO₃⁻ group, this deprotonation is one that occurs at physiological pH. In addition where a carboxyl group -COOH is deprotonated to give the anionic -COO⁻ group, this deprotonation is one that can occur at physiological pH. Moreover, charged salts of the molecules of the invention are encompassed. Saccharide rings can exist in an open and closed form, while closed forms are shown herein, open forms are also encompassed by the invention. Similarly, isomeric forms of the molecules of the invention are also encompassed, including tautomers, conformers, enantiomers and diastereoisomers, for example.

The counter-ions, which compensate the charged forms of the compounds of the present invention, are pharmaceutically acceptable counter-ions such as hydrogen, or more preferably alkali or alkali-earth metals ions, which include sodium, calcium, magnesium and potassium.

Other 'compound' group definitions will be readily understandable by the skilled person based on the previous definitions and the usual conventions of nomenclature.

Certain compounds of the invention exist in various regioisomeric, enantiomeric, tautomeric and diastereomeric forms. It will be understood that the invention comprehends the different regioisomers, enantiomers, tautomers and diastereomers in isolation from each other as well as mixtures.

It will be appreciated that any optional feature that has been described above in relation to any one aspect of the invention may also be applicable to any other aspect of the invention.

### GENERAL METHODS

The present invention will now be described in more detail, by way of the following non-limiting methods that can be used for synthesising the compounds of the present invention. The skilled person will, however, appreciate that these methods merely illustrate the present invention and in no way restrict its scope.

### Method A: Preparation of the alkylated protein

Methods of preparation of alkylated proteins are well known in the art. For example, Francis, M. B. et al. J. Am. Chem. Soc. 2006, 128, 1080-1081 discloses a method of preparing an alkylated compound akin to that of compound 1, below.

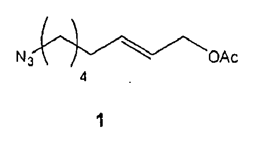

To a 1.5 mL eppendorf tube was added a 2 mM aqueous solution of protein (1 equiv.), a 14 mM aqueous solution of 1 (5 equiv.), an aqueous solution of the catalyst mixture (7 mM in Pd(OAc)₂, 1:12 Pd: TPPTS; final concentration = 40 µM Pd(OAc)₂, 0.2 equiv.; 480 µM TPPTS, 2.4 equiv.) and a 100 mM triethylammonium carbonate or phosphate buffer (pH 8.6, 243 µM). The reaction mixture was incubated at room temperature for 45 minutes and then applied to a NAP-5 gel filtration column that had been equilibrated with 3 column lengths of ddH₂O. 500 µL of the protein reaction mixture was eluted with 1 mL of ddH₂O to yield a solution of alkylated protein.

To determine the level of conversion, a sample of the alkylated protein was analysed using either ESI-MS or RP-HPLC (C18) which separated the unmodified and the singly modified protein components. Each species (i.e. the unmodified and the singly modified protein component) was readily identifiable by its UV spectrum.

### Method B: Ligation between the pegylated pentasaccharide 4 and the alkylated protein using a cycloaddition reaction is discussed below.

A protein alkylated as described in 'Method A: Preparation of the alkylated protein' (above) (200 µg, 0.5 mg/mL) and the pegylated pentasaccharide **4** (40 equiv.) were added to a solution of tris(1-benzyl-1*H*-(1,2,3)triazol-4-ylmethyl)-amine (2 mM) and tris(carboxyethyl)phospine (2 mM) in a mixture of 0.1M phosphate buffer (pH 8, 95 µL) and *tert-*butanol (5 µL) at 4°C. CuSO₄ was then added to a concentration of 1 mM.

Following incubation at 4 °C for 16 hours, the mixture was purified by passage through a High Performance Size Exclusion Chromatography (HPSEC). The molecular weight of the proteins was assessed by matrix-assisted laser desorption/ionization mass spectrometry (MALDI-MS) applying a high resolution time-of-flight (TOF).

### Method C: Ligation by reductive amination

A PEGylated pentasaccharide **8** was added to 15 mg of protein in 3.0 mL of buffer (50 mM potassium phosphate, pH 6.5) in a molar ratio of 5 moles of reagent per one mole of protein. To the reaction mixture, 10% (V/V) of 0.5 M sodium cyanoborohydride solution in water was added and stirred for 4 hours at room temperature. Separation of the unmodified protein and the different PEGylated species can be achieved by size exclusion chromatography (HPSEC). The content and the purity of the different species obtained were evaluated on SDS PAGE. The molecular weight of the proteins was assessed by matrix-assisted laser desorption/ionization mass spectrometry (MALDI-MS) applying a high resolution time-of-flight (TOF).

### Method D: Ligation by acylation

To a protein (5 mg/mL) in 50 mM sodium phosphate (pH 6.5) was added NHS-PEGylated pentasaccharide **9** (4.2 equiv.). After 70 min, the reaction was quenched with 1 M glycine. Quenched reaction products were dialysed against 100 mM sodium phosphate, 150 mM NaCl, pH 5.0 at 5 °C. Mono-PEGylated protein was purified from dialysates by Size Exclusion Chromatography (SEC).

The content and the purity of the different species obtained were evaluated on SDS-PAGE. The exact molecular weight of the proteins was assessed by matrix-assisted laser desorption/ionization mass spectrometry (MALDI-MS) applying a high resolution time-of-flight (TOF).

### Method E: Ligation by thiol-maleimide coupling

A protein or a Cys-incorporated protein was incubated at room temperature with Tris[2-carboxyethylphosphine] hydrochloride (TCEP, Pierce Chemical Company) (5 equiv.) and Maleimide-PEGylated pentasaccharide **10** (2 equiv.) at pH 8.5 for 60 minutes. The PEGylation reaction was then diluted 10-fold with 20 mM MES, pH 5.0, adjusted to pH 3.0, and subjected to a Size Exclusion Chromatography (SEC). The content and the purity of the different species obtained were evaluated on SDS-PAGE. The exact molecular weight of the proteins was assessed by matrix-assisted laser desorption/ionization mass spectrometry (MALDI-MS) applying a high resolution time-of-flight (TOF) in the positive ion mode.

Methods of preparing the intermediates of the present invention are shown below. Once synthesised, the intermediates can be reacted with an AT-binding oligosaccharide, for example, to yield an AT-binding oligosaccharide linker complex. The AT-binding oligosaccharide linker complex can then be reacted further with a protein molecule, as discussed above.

### Preparation of Intermediates

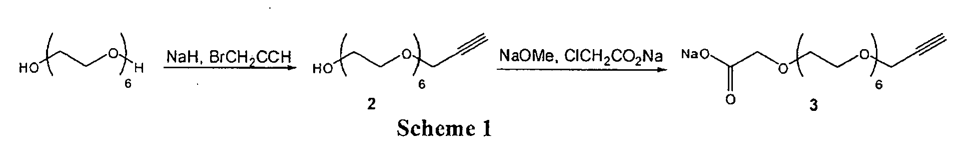

To a solution of hexaethylene glycol (500 mg, 1.7 mmol) in THF (8 mL) was added sodium hydride, 60% dispersion in grease (32 mg, 0.8 equiv.). The suspension was stirred for 45 min before a solution of propargyl bromide (166 mg, 0.82 equiv.) in THF (4 mL) was slowly added over 30 minutes. The mixture was stirred for 16 hours then quenched with an aqueous saturated solution of NH₄Cl, and then the organic phase was extracted with Et₂O, washed with water, brine, dried over Na₂SO₄, filtrated and finally concentrated *in vacuo.* The residue was chromatographed (Hexanes/EtOAc 3:2, 1:1, 2:3) to afford Monopropargyl-HEG 2 (163 mg, 30%) and the starting bromide.

Mass: CI method, negative mod: chemical mass = 320; experimental mass = 321.

To a methanol solution containing 0.44 mmol sodium methoxide, monopropargyl-HEG 2 (160 mg, 1.1 equiv.) was added. The solution was stirred for 15 minutes at 40 °C and methanol was then evaporated *in vacuo.* The residue was diluted with three volumes of dioxane and stirred with powered sodium chloroacetate (52 mg, 0.45 mmol) for 24 hours at reflux temperature. From the viscous solution resulted, dioxane was distilled off and the residue was redissolved in isopropyl alcohol and sodium chloride was separated by filtration. After removal of solvent, crude sodium salt of carbomethoxy-propargyl-HEG **3** was obtained (64 mg, 38%) from the filtrate as a viscous liquid.
Mass: CI method, negative mod: chemical mass = 384; experimental mass = 385.

The above synthetic steps are akin to those disclosed in Matsushima K. Tetrahedron Letters, 1979, 36, 3445-3448.

### PEGylated pentasaccharide 4

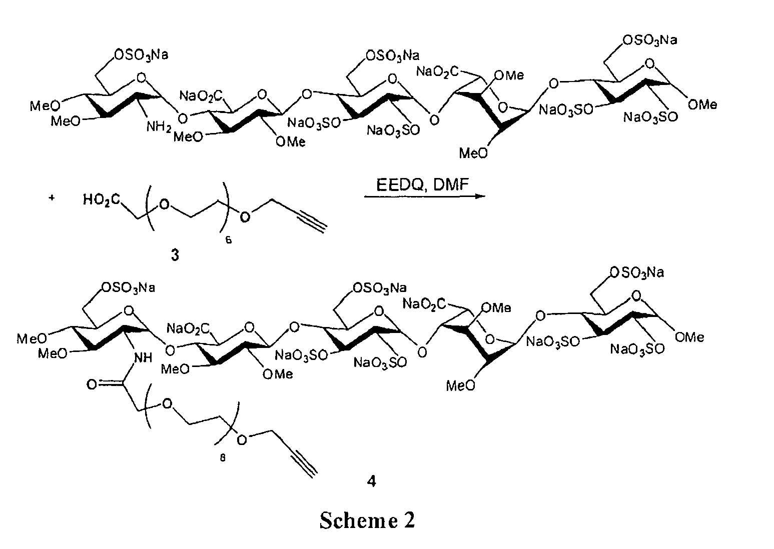

To a dry, stirred solution of a pentasaccharide (20 mg, 0.013 mmol) (cf. above and WO 99/25720A1 and WO 02/24754) and carbomethoxy-propargyl-HEG **3** (5 mg, 1.1 equiv.) in DMF (10 mL), ethyl-2-ethoxy-1,2-dihydroquinoline-1-carboxylate (EEDQ) (3 mg, 1.1 equiv.) was added and the reaction was left under an atmosphere of nitrogen and at room temperature for 24 hours. The reaction mixture was deposited on a Sephadex G25F (160 mL) column eluted with NaCl (0.2 M). The fractions comprising the product are combined, concentrated and desalted on a Sephadex G25F (160 mL) eluted with water. After lyophilising, a pegylated pentasaccharide **4** (12 mg, 50%) was obtained. Mass: ESI method, negative mod: chemical mass = 1871; experimental mass = 1872.

The above synthetic scheme is akin to one disclosed in Chernyak, A. Y. et al. Carbohydrate Res., 1992, 223, 303-309.

### PEGylated pentasaccharide 8

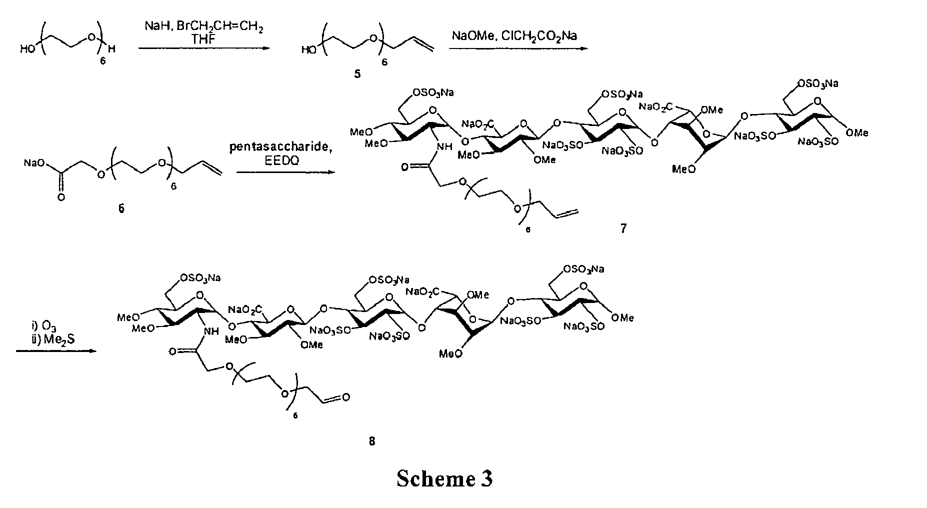

To a solution of hexaethylene glycol (500 mg, 1.7 mmol) in THF (8 mL) was added sodium hydride, 60% dispersion in grease (32 mg, 0.8 equiv.). The suspension was stirred for 45 min before a solution of allyl bromide (120 µL, 0.82 equiv.) in THF (2 mL) was slowly added over 20 minutes. The mixture was stirred for 16 hours, then quenched with an aqueous saturated solution of NH₄Cl, and then the organic phase was extracted with Et₂O, washed with water, brine, dried over Na₂SO₄, filtrated and finally concentrated *in vacuo.* The residue was chromatographed (Hexanes/EtOAc 3:2, 1:1, 2:3) to afford Mono-allyl-HEG 5 (164 mg, 30%) and the starting bromide.
NMR (CDCl₃) 5.65 ppm (m, 1H), 5.01 ppm (dd, 2H, *J* = 6.4, 13.6 Hz), 3.5 ppm (s, -OCH₂CH₂-O-).
Mass: CI method, negative mod: chemical mass = 322; experimental mass = 323.

To a methanol solution containing 0.44 mmol sodium methoxide, Mono-allyl-HEG **5** (161 mg, 1.1 equiv.) was added. The solution was stirred for 15 minutes at 40°C and methanol was then evaporated *in vacuo.* The residue was diluted with three volumes of dioxane and stirred with powered sodium chloroacetate (52 mg, 0.45 mmol) for 24 hours at reflux temperature. From the viscous solution resulted, dioxane was distilled off and the residue was redissolved in isopropyl alcohol and sodium bromide was separated by filtration. After removal of solvent, a crude sodium salt of carbomethoxy-propargyl-HEG 6 (67 mg, 38%) was obtained from the filtrate as a viscous liquid. Mass: CI method, negative mod: chemical mass = 380; experimental mass = 381.

The above synthetic steps are akin to those disclosed in Matsushima K. Tetrahedron Letters 1979, 36, 3445-3448.

### PEGylated pentasaccharide 7

To a dry, stirred solution of a pentasaccharide (20 mg, 0.013 mmol) (cf. scheme 2 above) and carbomethoxy-allyl-HEG **6** (6 mg, 1.1 equiv.) in DMF (2 mL), ethyl-2-ethoxy-1,2-dihydroquinoline-1-carboxylate (EEDQ) (3 mg, 1.1 equiv.) was added and the reaction was left under an atmosphere of nitrogen and at room temperature for 24 hours. The reaction mixture was deposited on a Sephadex G25F (160 mL) column, which was eluted with NaCl (0.2 M). The fractions comprising the product were combined, concentrated and desalted on a Sephadex G25F (160 mL), which was eluted with water. After lyophilising, a pegylated pentasaccharide 7 (12 mg, 50%) was obtained.
Mass: ESI method, negative mod: chemical mass = 1889; experimental mass = 1890.

### PEGylated pentasaccharide-aldehyde 8

Compound 7 (10 mg, 5.29 µmol) was solubilized in a mixture of CH₂Cl₂/MeOH:1/1 (2 mL) and cooled to -78 °C before ozone was bubbled in the solution. After 30 minutes, a light blue colour persisted and the ozone flow was stopped and replaced by argon until the solution turned colourless. Then, Me₂S (1 µL, 3 equiv.) was added, and the resulting mixture was allowed to reach 0 °C and then stirred for 1 hours. The reaction mixture was deposited on a Sephadex G25F (160 mL) column eluted with NaCl (0.2 M). The fractions comprising the product are combined, concentrated and desalted on a Sephadex G25F (160 mL) eluted with water. After lyophilising, carbomethoxy-HEG-aldehyde **8** (6.5 mg, 65%) was obtained.
NMR (CD₃OD) 3.5 ppm (s, -OCH₂CH₂-O-), 9.66 ppm(s, -CHO)-; Mass: CI method, negative mod: chemical mass = 1891 ; experimental mass = 1892.

### PEGylated pentasaccharide 9

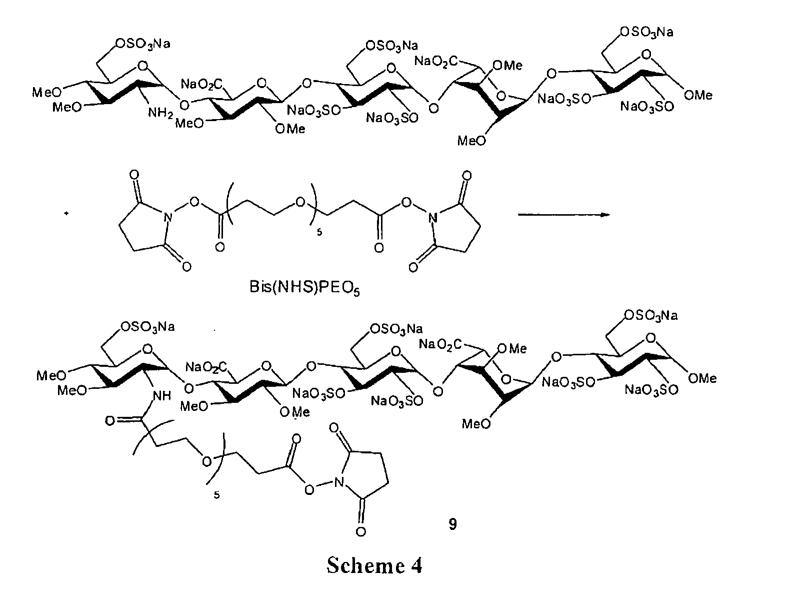

To a dry, stirred solution of a pentasaccharide (20 mg, 0.013 mmol), shown above, in 0.5% sodium hydrogen carbonate (2 mL) is added Bis(NHS)PEO₅ (Pierce Chemical Company) (7 mg, 1.1 equiv.). After stirring for 16 hours at room temperature, the reaction mixture was deposited on a column Sephadex G-25F, which was eluted with sodium chloride. The fractions comprising the product were concentrated and desalting was carried out on the same column, which was eluted with water. After lyophilizing, 11 mg of NHS-PEGylated pentasaccharide **9** (45%) was obtained.
Mass: ES1 method, negative mod: chemical mass = 1922; experimental mass = 1923.

### PEGylated pentasaccharide 10

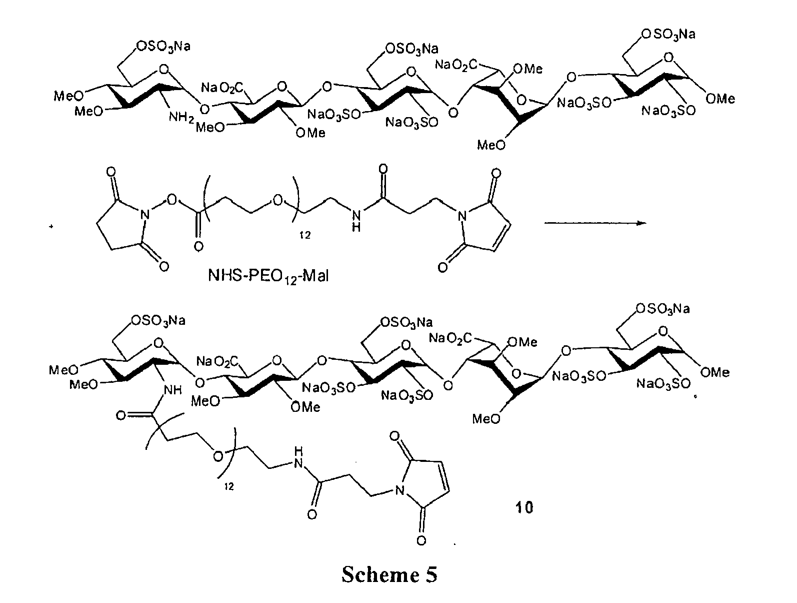

To a dry, stirred solution of a pentasaccharide (20 mg, 0.013 mmol), shown above, in 0.5% sodium hydrogen carbonate (2 mL) is added NHS-PEO₁₂-Mal (Pierce Chemical Company) (12 mg, 1.1 equiv.). After stirring for 16 hours at room temperature, the reaction mixture was deposited on a column Sephadex G-25F, which was eluted with sodium chloride (0.2 M). The fractions comprising the product were concentrated and desalting was carried out on the same column, which was eluted with water. After lyophilizing, 12 mg of NHS-PEGylated pentasaccharide **10** (43%) was obtained.
Mass: ESI method, negative mod: chemical mass = 2256; experimental mass = 2257.

### Examples

From the above general methods, it will be apparent to the skilled person that proteins can be joined to an AT-binding oligosaccharide-linker complex. However, for completeness, the present invention will be further illustrated by the following non-limiting specific examples. Again, it will be apparent to the skilled person that these specific examples merely illustrate the present invention and other proteins and linkers, for example, could be used.

### PEGylated enfuvirtide

Enfuvirtide (50 mg, 11.1 µmol) is treated according to the 'Method A: Preparation of the alkylated protein' (above) to give alkylated enfuvirtide **11** (24 mg, 47%).
Mass: method ESI, negative mod: calculated mass = 4643; experimental mass = 4644.

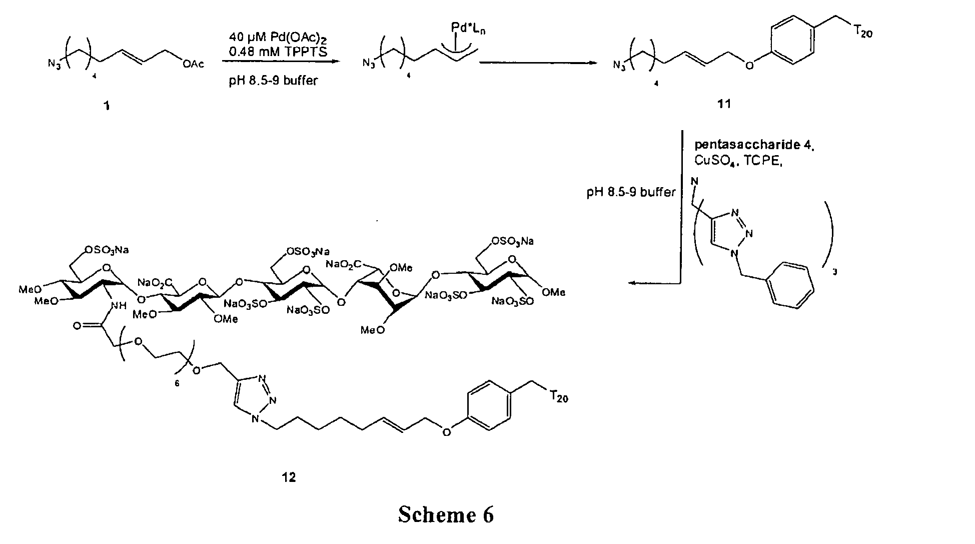

Alkylated enfuvirtide **11** (24 mg, 5.1 µmol) was treated according to 'Method B: Ligation between the pegylated pentasaccharide and the alkylated protein' (above) to give pegylated enfuvirtide **12** (7.6 mg, 23%).
Mass: MALDI-MS: 6516 [M+H]⁺.

An alternative specific example of pegylated enfuvirtide is shown below. 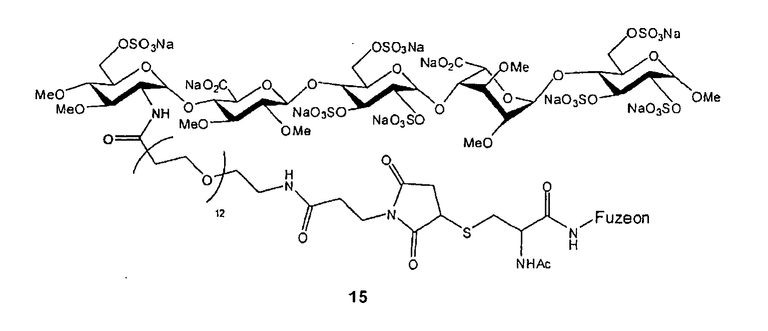 Cys-enfuvirtide (10 mg, 2 µmol) was treated according to 'Method E: Ligation by thiol-maleimide coupling' (above) to give pegylated Cys-enfuvirtide **15** (8 mg, 59%).
Mass: MALDI-MS: 6806 [M+ H]⁺.

### PEGvlated Erythropoietin (EPO)

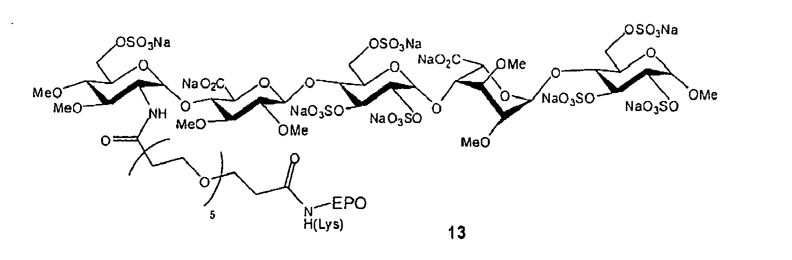

EPO (10 mg, 335 nmol) was treated according to 'Method D: Ligation by acylation' (above) to give pegylated EPO **13** (2.9 mg, 28%).
Mass: MALDI-MS: 31614 [M+ H]⁺.

### PEGylated IFN-α2

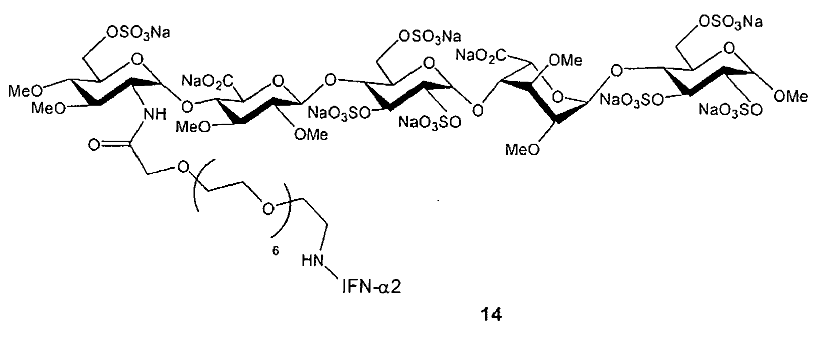

IFN-α2 (10 mg, 519 nmol) is treated according to 'Method C: Ligation by reductive amination' (above) to give pegylated IFN-α2 **14** (2.9 mg, 27%).
Mass: MALDI-MS: 21143 [M+ H]⁺.

### BIOLOGICAL TESTING

It will be understood that a variety of assays are suitable for testing the biological activity of the compounds of the present invention and that proteins can be assayed by using an assay that is suitable for that particular protein. However, suitable assays for testing the biological activity of enfuvirtide, EPO and IFN-α are listed below.

### Enfuvirtide

### Virus-free cell fusion assay

This assay was performed using either HeLa-CD4-LTR β-gal or U373-MAGI (Multinuclear Activation of a Galactosidase Indicator) cells expressing CD4 constitutively and β-galactosidase under the control of the HIV-1 LTR promoter; the U373-MAGI-CXCR4 (expressing in addition the CXCR4 gene); or the U373-MAGI-CCR5 (expressing in addition the CCR5 gene). Cells constitutively expressing the HIV-1 tat and GP160 (called HL160tat cells) were used because they express viral proteins that allow fusion with the HeLa or U373 cells. The tat protein will switch on the LTR-driven β-galactosidase gene expression if the fusion occurs.

HeLa or U373 and HL160tat cells were co-cultivated in DMEM+2%FBS and incubated at 37 °C in 5% CO₂ in presence of 1/3 serial concentrations (ranging from 0.0045 to 10 µg/ml) of the compounds of the present invention for 24-48 hours.

The cells were fixed and the β-galactosidase reporter gene was detected with the X-gal substrate in case of fusion. The number of syncytia was counted and the IC50 value was defined as the dilution that resulted in a 50% reduction of the syncytia formation.

The anti fusion activity of the invention was maintained as compared to that of enfuvirtide.

### Anti-HIV-1 activity on MT-4 cells

MT-4 cells were seeded in presence of the invention and diluted HIV-1. Cytopathic effects induced by the virus were checked regularly microscopically. After 4 days of infection, the cell viability was assessed spectrophotometrically using the MTT assay. The median inhibitory concentration (IC50) was calculated from each dose-response curve.

The ability of the compounds of the present invention to decrease the cytopathic effect induced by the virus HIV-1 on MT-4 cells was then observed.

### EPO

### UT7 cell proliferation assay

The *in vitro* proliferative activity was assessed in human UT7 (acute myeloid leukaemia) cells.

UT7 cells were washed in α-MEM and starved in α-MEM with added 2 mM L-glutamine and 5% FBS for 4 hours. Then, the UT7 cells were washed in α-MEM, counted and suspended at 2x10⁵ cells/ml in α-MEM + 10% FBS + Penicillin / streptomycin 1 % + 2 mM L-glutamine. A stock solution of the compounds of the present invention was diluted and followed by 1:2 serial dilutions in α-MEM + 10% FBS + Penicillin / streptomycin 1% + 2 mM L-glutamine. The UT7 cell suspension and various dilutions of the compounds ranging from 0 to 2.7x10⁻¹⁰ M of the present invention were mixed 1/1 v/v, plated and incubated at 37 °C in 5% CO₂ for approximately 68 hours.

The cell proliferation assay was assessed by adding Seroctec's Alamarblue reagent (10 µl). After 4-5 hours of incubation the optical density of the assay was measured.

The ability of the compounds of the present invention to induce UT7 cell proliferation was then observed.

### Clonogenic assay

The biological activity of the compounds of the present invention was assessed by the BFU-E clonogenic assay, which is well known in the art. Peripheral blood mononuclear cells (PBMC) were plated in methylcellulose-based medium containing FBS, rhuIL3 and SCF. The concentration of the compounds of the present invention varied from 0 to 5.48x 10⁻¹⁰ M.

The PBMC cells were plated in 35 mm Petri dishes and incubated in a fully humidified atmosphere with 5% of CO₂ at 37 °C for 14 days.

The ability of the compounds of the present invention to enhance erythroid colony formation was then observed.

### IFN-α

### In vitro anti-proliferative activity in human tumor cell line ACHN

The *in vitro* anti-proliferative activity of the compounds of the present invention was assessed in human ACHN (renal adenocarcinoma) cells.

ACHN cells were washed in EMEM, counted and suspended at 4x10⁴ cells/ml in EMEM + 4-10% FBS + Penicillin / streptomycin 1% + 2mM L-glutamine (complete medium). 50 µl of cell suspension (2,000 cells/well) were plated and allowed to adhere.

For each concentration tested (ranging from 0 to 20.4x10⁻¹⁰M), the compounds of the present invention were diluted in EMEM + Penicillin / streptomycin 1% + 2 mM L-glutamine at a two times concentration and 50 µl were added to the corresponding well.

The ACHN cells were incubated at 37 °C in 5% CO₂ for approximately 90 hours. The cell proliferation assay was then assessed by adding 3-(4,5-dimethylthiazol-2-yl)-2,5-diphenyltetrazolium (MTT). After 4-5 hours of incubation the optical density of the assay was measured.

The ability of the compounds of the present invention to reduce ACHN cell proliferation was then observed.

### Increase of MHC Class I molecules expression in MOLT-4 cells

The increase of MHC class I molecules expression was assessed in human MOLT-4 (acute lymphoblastic leukaemia) cells.

The MOLT-4 cells were washed in RPM11640 and starved in RPMI1640 with added L-glutamine overnight. Then, the MOLT-4 cells were washed in RPMI1640, counted and suspended at 3x10⁵ cells/ml in RPMI1640 + 10% FBS + Penicillin / streptomycin 1% + 2mM L-glutamine (compete medium) in presence of various concentrations, ranging from 0 to 5.2x10⁻¹⁰ M, of the compounds of the present invention.

The MOLT-4 cells were incubated at 37 °C in 5% CO₂ for 48 hours then stained with an antihuman HLA A,B,C from Serotec (0.1 µg/10⁵ cells) and washed. The expression of MHC class I was assessed by flow cytometry.

The ability of the compounds of the present invention to increase the expression of MHC class I was then observed.

### PHARMACOKINETICS

Solutions of the compounds of the present invention were prepared in Phosphate Buffered Saline (pH 7.4). These solutions were administered by the intravenous or subcutaneous routes that would, presumably, be the preferred routes of administration used in humans.

The pharmacokinetics of the compounds of the present invention was investigated in female Wistar rats after a single intravenous or subcutaneous administration using a LC-MS procedure. The blood of the animals was sampled on citrate tubes over a period of several days after the administration. The time to reach maximum plasma dose (Tₘₐₓ) and the maximum plasma dose (Cₘₐₓ) were determined from the plasma level *vs.* time data. The area under the curve *vs.* time curve up to ∞ (AUC_{0→∞}) after administration of the compounds of the present invention was calculated using the trapezoid rule. After administration of a single subcutaneous dose in rats, the compounds of the present invention were rapidly absorbed from the injection site, with peak plasma Tₘₐₓ occurring at several hours later and the maximum plasma dose Cₘₐₓ was measured at this time, in ng/ml. After administration of a single intravenous dose in rats, the AUC_{0→∞} was also measured, in µg.h/ml.

The inherent factor-Xa inhibitory activity of the compounds of the present invention was also assessed as a means of detection in a similar process to that described above. Again, the blood of the animals was sampled on citrate tubes over a period of several days after the administration. The time to reach maximum anti-Xa activity (Tₘₐₓ) and the maximum plasma anti-Xa activity ([aXa]max) was determined from the plasma anti-Xa activity *vs*. time data. The area under the plasma anti-Xa activity *vs.* time curve up to ∞ (AUC_{0→∞}) after administration of the compounds of the present invention was calculated using the trapezoid rule. After administration of a single subcutaneous dose in rats, the compounds of the present invention were rapidly absorbed from the injection site, with peak plasma anti-Xa activity Tₘₐₓ occurring several hours later and the maximum plasma anti-Xa activity [aXa]max was measured at this time, in ng/ml. After administration of a single intravenous dose in rats, the AUC_{0→∞} was also measured, in IU.h/ml. The apparent volume of distribution was small indicating restriction mainly to the blood compartment, as would be expected for the compounds of the present invention, which display a high and specific affinity for plasma anti-thrombin.

### Quantification of the carrier pentasaccharide in rat plasma

Rat plasmatic concentration of the carrier was determined by its anti-Factor Xa activity using COATEST® Heparin, which allows the specific determination of the anti-Factor Xa activity of heparin. The COATEST® Heparin Kit was from Chromogenix, Monptelliers. Absorbance measures were performed with the Multiskan Ascent absorbance plate reader (Thermo Labsystems (Helsinki, Finland). Normal rat plasma was obtained from Biopredict.

Rat blood (9 volumes) was mixed with sodium citrate (1 volume) and preferably cooled immediately on ice to minimise release of heparin antagonists from blood cells. As soon as possible after blood collection, the blood was subjected to a centrifuge at 2000 x g for 20 minutes at low temperature. A standard curve was prepared by a two-step procedure for dilution of the carrier. First, the carrier was diluted in physiological serum. Then, a 100-fold dilution was performed in working buffer (for example, Tris 50 mM, pH 8.4). Several carrier solutions were further diluted with antithrombin, normal rat plasma and different volumes of working buffer. After the standard curve construction, antithrombin and working buffer were added to the tested rat plasma samples and were incubated at 37 °C for 3 minutes. The dosage of anti-factor Xa activity was then performed by the mix of diluted test plasma or standard to factor Xa following by 30 seconds of incubation at 37 °C. The apparition of colour occurs after addition of 200 µl of chromogenic substrate S-2222.

The solution that was obtained was then mixed and incubated at 37 °C for exactly 3 minutes and the reaction was finally stopped with Acetic acid 20%. A blank was composed of 200 µl of sample blank, 300 µl of acetic acid and 300 µl of water and the absorbance was read at 405 nm. The respective blank activities for standard were subtracted from their absorbance at 405 nm. The corrected absorbencies were plotted against their concentrations of the carrier pentasaccharide. The obtained standard curve allows the calculation of unknown carrier concentration in plasma.

## Claims

1. A compound comprising a protein conjugated to an oligosaccharide, *via* a linker, wherein the oligosaccharide is capable of binding to antithrombin (AT);
or a salt, solvate or prodrug thereof.

2. The compound of claim 1 wherein the AT-binding oligosaccharide is of Formula (I): 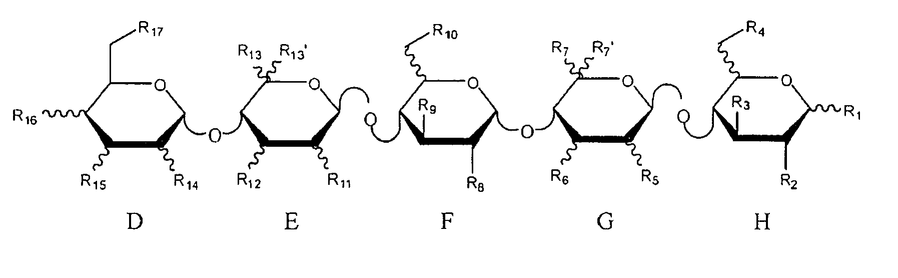 wherein:
R₁ is selected from the group consisting of: O-acyl, O-alkyl, O-alkenyl, O-alkynyl, O-alkylaryl, O-aryl and a monosaccharide unit of formula 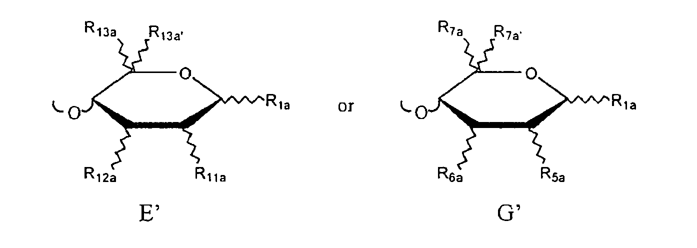 wherein R₁ₐ is selected from the group consisting of: O-acyl, O-alkyl, O-alkenyl, O-alkynyl, O-alkylaryl and O-aryl;
R₁₆ is selected from the group consisting of: OSO₃H, OH, O-acyl, O-alkyl, O-alkenyl, O-alkynyl, O-alkylaryl, O-aryl and a monosaccharide unit of formula 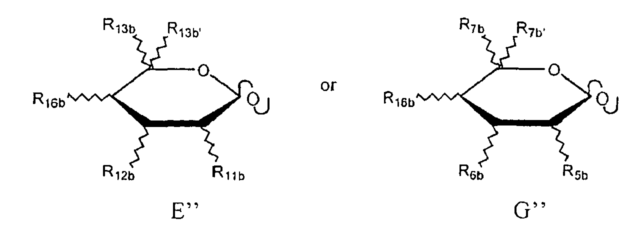 wherein R_{16b} is selected from the group consisting of: OSO₃H, OH, O-acyl, O-alkyl, O-alkenyl, O-alkynyl, O-alkylaryl and O-aryl;
R₂ and R₈ are each independently selected from the group consisting of: OSO₃H and NHSO₃H;
R₃, R₄, R₆, R₆ₐ, R_{6b}, R₁₀, R₁₁, R₁₁ₐ, R_{11b}, R₁₂, R₁₂ₐ and R_{12b} are each independently selected from the group consisting of: OSO₃H, O-acyl, O-alkyl, O-alkenyl, O-alkynyl, O-alkylaryl, and O-aryl;
R₅, R₅ₐ and R_{5b} are each independently selected from the group consisting of: OSO₃H, O-acyl, O-alkyl, O-alkenyl, O-alkynyl, O-alkylaryl and O-aryl;
R₇, R₇ₐ and R_{7b} are COOH;
R_{7'}, R_{7a'} and R_{7b'} are H;
alternatively, R₅ together with R_{7'}, R₅ₐ together with R_{7a'}, and R_{5b} together with R_{7b'} may each independently be selected from the group consisting of: O-CH₂ and O-CH₂CH₂ whereby, together with the carbon atoms to which they are attached, they form a heterocyclic ring;
R₉ and R₁₇ are each OSO₃H;
R₁₃, R₁₃ₐ and R_{13b} are each COOH;
R_{13'}, R_{13a'} and R_{13b'} are each independently selected from the group consisting of: H and alkyl;
R₁₄ is selected from the group consisting of: OSO₃H, NHSO₃H, OH, O-acyl, O-alkyl,
O-alkenyl, O-alkynyl, O-alkylaryl and O-aryl; and
R₁₅ is selected from the group consisting of: OSO₃H, OH, O-acyl, O-alkyl, O-alkenyl, O-alkynyl, O-alkylaryl and O-aryl;
provided that at least one of the groups R₁, R₁ₐ, R₄, R₁₄, R₁₅, R₁₆ and R_{16b} is independently selected from the group consisting of: an amine, an oxygen atom and a carboxylic acid and is conjugated to the linker;
or a salt, solvate or prodrug thereof.

3. The compound, salt, solvate or prodrug of claim 2 wherein the AT-binding oligosaccharide of Formula (I) is a pentasaccharide.

4. The compound, salt solvate or prodrug of any one of the claims 2 and 3 wherein R_{13'}, R_{13a'} and R_{13b'} are independently selected from the group consisting of: hydrogen and ethyl.

5. The compound, salt, solvate or prodrug of any one of the claims 2 to 4 wherein the monosaccharide units D, E, E', E", F and H of the AT-binding oligosaccharide have the D-gluco stereochemistry as follows:

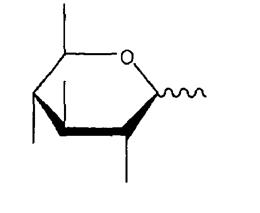

6. The compound, salt, solvate or prodrug of any one of the claims 2 to 5 wherein the monosaccharide unit G, G' and G'' of the AT-binding oligosaccharide have the L-ido stereochemistry as follows:

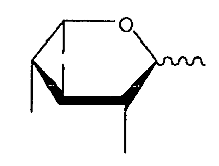

7. The compound, salt, solvate or prodrug of any one of the claims 2 to 6 wherein R₅ together with R_{7'} may each independently selected from the group consisting of: O-CH₂ and O-CH₂CH₂ whereby, together with the carbon atoms to which they are attached, they form a heterocyclic ring.

8. The compound, salt, solvate or prodrug of any one of the claims 2 to 7 wherein R₃ is OSO₃H.

9. The compound, salt, solvate or prodrug of any one of the claims 2 to 8 wherein the linker is conjugated to the AT-binding oligosaccharide through the group at the R₁₄ position.

10. The compound, salt, solvate or prodrug of claim 9 wherein R₁₄ is NH.

11. The compound, salt, solvate or prodrug of any one of the claims 2 to 10 wherein:
R₁, R₅, R₆, R₁₁, R₁₂, R₁₅, R₁₆ are each O-(C₁₋₆ alkyl);
R₂, R₃, R₄, R₈, R₉, R₁₀ and R₁₇ are each OSO₃H; and
R₁₃ is H;
R₁₄ is NH.

12. The compound, salt, solvate or prodrug of any one of the claims 2 to 11 wherein:
R₁, R₅, R₆, R₁₁, R₁₂, R₁₅, R₁₆ are each O-CH₃.

13. The compound, salt, solvate or prodrug of any one of the claims 2 to 8 wherein the linker is conjugated to the AT-binding oligosaccharide through the group at the R₁ position.

14. The compound, salt, solvate or prodrug of claim 13 wherein R₁ is independently selected from the group consisting of: an amine, an oxygen atom and a carboxylic acid and is conjugated to the linker.

15. The compound, salt, solvate or prodrug of any one of the claims 2 to 8 wherein the linker is conjugated to the AT-binding oligosaccharide through the group at the R₁₆ position.

16. The compound, salt, solvate or prodrug of claim 15 wherein R₁₆ is independently selected from the group consisting of: an amine, an oxygen atom and a carboxylic acid and is conjugated to the linker.

17. The compound, salt, solvate or prodrug of any one of the claims 2 to 8 wherein the linker is conjugated to the AT-binding oligosaccharide through the group at the R₄ position.

18. The compound, salt, solvate or prodrug of claim 17 wherein R₄ is independently selected from the group consisting of: an amine, an oxygen atom and a carboxylic acid and is conjugated to the linker.

19. The compound, salt, solvate or prodrug of claim 1 wherein the AT-binding oligosaccharide is of the formula:

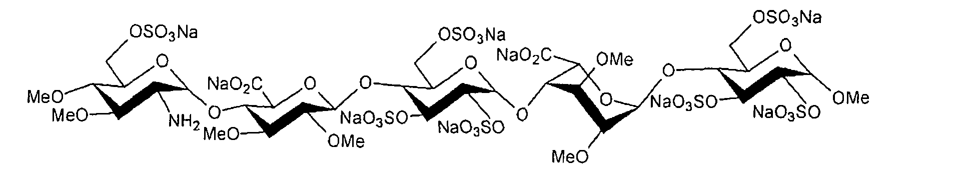

20. The compound, salt, solvate or prodrug of any one of the claims 1 to 19 wherein the linker is a hydrocarbon chain optionally substituted with, or interrupted with, one or more heteroatoms.

21. The compound, salt, solvate or prodrug of claim 20 wherein linker comprises a chain of from 6 to 250 atoms.

22. The compound, salt, solvate or prodrug of any one of the claims 20 to 21 wherein the linker is conjugated to the AT-binding oligosaccharide through any one of the following bonds: amine, amide, ester and ether.

23. The compound, salt, solvate or prodrug of any one of the claims 20 to 22 wherein the linker is conjugated to the protein through any one of the following bonds: amine, amide, ether, ester and thioether.

24. The compound, salt, solvate or prodrug of any one of the claims 20 and 23 wherein the linker further comprises at least one ethylene glycol monomer.

25. The compound, salt, solvate or prodrug of any one of the claims 20 and 24 wherein the linker further comprises at least one amino acid.

26. The compound, salt, solvate or prodrug of any one of the claims 20 and wherein the linker is of formula II: 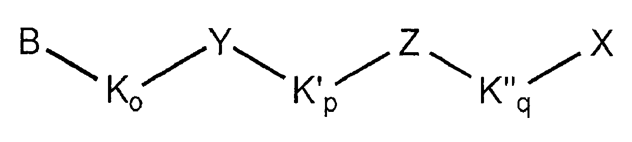 wherein:
B and X are both organic moieties, wherein the linker is conjugated to the AT-binding oligosaccharide *via* B and to the protein *via* X;
K, K', K" are independently selected from the group consisting of: an amino acid, an ethylene glycol monomer, alkyl , alkenyl and alkoxy;
o, p and q are integers independently selected from 0 to 40;
or Kₒ, K'ₚ and K''_{q} are independently a chain of amino acids that comprises from 2 to 40 amino acids, preferably from 3 to 10 amino acids, more preferably from 4 to 8 amino acids and more preferably from 5 to 7 amino acids;
Z is not present or is independently selected from the group consisting of: heterocyclyl and heteroaryl;
Y is not present or is independently selected from the group consisting of: a nitrogen atom, a carbon atom and a phosphorous atom optionally substituted with one or more groups of Formula IIa: 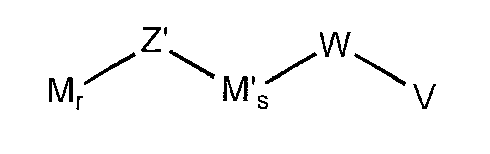 wherein M and M' are independently selected from the group consisting of: an amino acid, a chain of amino acids, an ethylene glycol monomer, alkyl, alkenyl and alkoxy;
r and s are integers independently selected from 0 to 40;
or M and M' are independently a chain of amino acids that comprises from 2 to 40 amino acids, preferably from 3 to 10 amino acids, more preferably from 4 to 8 amino acids and more preferably from 5 to 7 amino acids;
Z' is not present or is independently selected from the group consisting of: heterocyclyl and heteroaryl;
W is an organic moiety through which the linker is conjugated to a protein;
V is a protein; and
wherein when any of K, K', K", M and M' are alkyl they are independently optionally substituted and/or interrupted with one or more heteroatoms.

27. The compound, salt, solvate or prodrug of claim 26 wherein when any of K, K', K", M and M' are alkyl they are independently optionally substituted and/or interrupted with one or more atoms independently selected from the group consisting of: oxygen, nitrogen and sulphur.

28. The compound, salt, solvate or prodrug of any one of the claim 26 and 27 wherein Z is independently selected from the group consisting of heterocyclyl and heteroaryl and Y is not present.

29. The compound, salt, solvate or prodrug of any one of the claims 26 to 28 wherein K, K', K", M and M' are ethylene glycol monomers.

30. The compound, salt, solvate or prodrug of any one of the claims 26 to 29 wherein Z and Z' are independently selected from the group consisting of: triazolyl and succinimidyl.

31. The compound, salt, solvate or prodrug of any one of the claims 26 to 30 wherein:
Z and Y are both not present; and
p and q are both 0.

32. The compound, salt, solvate or prodrug of any one of the claims 26 to 31 wherein B is selected from the group consisting of: alkyl, alkenyl, alkynyl, aryl, heteroaryl, heterocyclyl, aminoalkyl, O, R_{A}-O, R_{A}-O-R_{A}, COO, R_{A}-COO, R_{A}-COO-R_{A}, S, R_{A}-S, R_{A}-S-R_{A}, SO₂ R_{A}-SO₂, R_{A}-SO₂-R_{A}, SO₃H R_{A}-SO₃H, OSO₃H, R_{A}-OSO₃H, NH, R_{A}-NH, R_{A}-NH-R_{A}, N(R_{A})₂, R_{A}-N(R_{A})₂, NHC(O), R_{A}-NHC(O), R_{A}-NHC(O)R_{A}, C(O)NH, R_{A}-C(O)NH, R_{A}-C(O)NH-R_{A}, C(O)N(R_{A})₂, R_{A}-C(O)N(R_{A})₂, N(R_{A})₂, R_{A}-N(R_{A})₂, C(O), R_{A}-C(O), R_{A}-C(O)-R_{A}, wherein R_{A} is selected from the group consisting of: alkyl, alkenyl, alkynyl, cycloalkyl, alkoxyalkyl; and
wherein any of B and R_{A} are independently optionally substituted with one or more groups independently selected from COOH, COO(alkyl), SH, S(alkyl), SO₂H, SO₂(alkyl), SO₂(aryl), SO₂(alkaryl), P(OH)(O)₂, halo, halo(alkyl), perhalo(alkyl), OH, O(alkyl), =O, NH₂, =NH, NH(alkyl), N(alkyl)₂, =N(alkyl), NHC(O)(alkyl), C(O)NH₂, C(O)NH(alkyl), C(O)N(alkyl)₂, C(O)NH(aryl), C(O)N(aryl)₂, C(O)NH(aralkyl), C(O)N(aralkyl)₂, C(O)NH(alkaryl), C(O)N(alkaryl)₂, NO₂, CN, SO₂, SO₂NH₂, C(O)H and C(O)(alkyl), alkyl, alkoxyalkyl, alkoxyaryl, alkynyl, aralkyl, alkaryl, heterocyclyl, heteroaryl and aryl.

33. The compound, salt, solvate or prodrug of claim 32 wherein B is selected from the group consisting of: methyl, ethyl, propyl, butyl, hexyl, heptyl, cyclohexyl, cycloheptyl, cyclooctyl, cyclononyl, cyclodecyl, cycloundecyl, tetramethylcyclohexyl, cyclohexylmethyl, hydroxyacetamide, acetamide, methylacetamide, ethylacetamide, propylacetamide, butylacetamide, hexylacetamide, heptylacetamide, amino, 2-amino ethanol, methylamine, ethylamine, propylamine, butylamine, methanol, ethanol, propanol, butanol, heptanol, methylaldehyde, ethylaldehyde, propylaldehyde, butylaldehyde, hydroxylethanal, hydroxypropanal, hydroxylbutanal, phenyl and methyl any of which are optionally substituted with one or more groups independently selected from =O, COOH, SH, SO₂H, P(OH)(O)₂, halo, trihalomethyl, OH, NH₂, =NH, NH(alkyl), =N(alkyl), NO₂ CN, OCH₃, SO₂NH₂ and C(O)H, alkyl, alkoxyalkyl, alkoxyaryl, aralkyl, alkaryl, heterocyclyl, heteroaryl and aryl.

34. The compound, salt, solvate or prodrug of claim 33 wherein B is selected from the group consisting of: -O-, -NH-, -C(O)CH₂-, -C(O)-, -C(O)CH₂O-, phenyl and methyl.

35. The compound, salt, solvate or prodrug of any one of the claims 26 to 34 wherein X and W are independently selected from the group consisting of: alkyl, alkenyl, alkynyl, alkoxyalkyl, cycloalkyl, aryl, heteroaryl, R_{A}-heteroaryl, R_{A}-heteroaryl-R_{A}, aminoalkyl, heterocyclyl, R_{A}-heterocyclyl, R_{A}-heterocyclyl-R_{A}, COO, R_{A}-COO, R_{A}-COO-R_{A}, C(O), R_{A}-C(O), R_{A}-C(O)-R_{A}, NHC(O), R_{A}-NHC(O), R_{A}-NHC(O)-R_{A}, C(O)NH, R_{A}-C(O)NH, R_{A}-C(O)NH-R_{A}, R_{A}-NH, R_{A}-NH-R_{A}, succinimide, R_{A}-succinimide, R_{A}-succinimide-R_{A}, imidazole, R_{A}-imidazole, R_{A}-imidazole-R_{A}, maleimide, R_{A}-maleimide, R_{A}-maleimide-R_{A},
wherein R_{A} is independently selected from the group consisting of: alkyl, alkenyl, alkynyl, alkoxy, alkenyloxy, alkenyloxytolyl, alkenyloxyaryl, and
wherein any of the groups are optionally substituted with one or more groups independently selected from COOH, COO(alkyl), SH, S(alkyl), SO₂H, SO₂(alkyl), SO₂(aryl), SO₂(alkaryl), P(OH)(O)₂, halo, halo(alkyl), perhalo(alkyl), OH, O(alkyl), =O, NH₂, =NH, NH(alkyl), N(alkyl)₂, =N(alkyl), NHC(O)(alkyl), C(O)NH₂, C(O)NH(alkyl), C(O)N(alkyl)₂, C(O)NH(aryl), C(O)N(aryl)₂, C(O)NH(aralkyl), C(O)N(aralkyl)₂, C(O)NH(alkaryl), C(O)N(alkaryl)₂, NO₂, CN, SO₂, SO₂NH₂, C(O)H and C(O)(alkyl), alkyl, alkoxyalkyl, alkoxyaryl, alkynyl, aralkyl, alkaryl, heterocyclyl, heteroaryl and aryl.

36. The compound, salt, solvate or prodrug of claim 35 wherein X and W are independently selected from the group consisting of: methyl, ethyl, propyl, butyl, hexyl, heptyl, methylamine, ethylamine, propylamine, butylamine, hexylamine, heptylamine, ethene, propene, formaldehyde, cyclohexyl, cycloheptyl, phenyl, benzyl, cyclohexyl, cycloheptyl, cyclooctyl, cyclononyl, cyclodecyl, cycloundecyl, tolyl, ethylbenzyl, xylyl, isopropylbenzyl, cyclohexylmethyl, methoxyphenyl, phenethyl, butylphenyl, propylbenzyl, mesitylyl, ethyltolyl, butylbenzyl, diethylbenzyl, dimethylethylbenzyl, tetramethylbenzyl, dipropylbenzyl, triethylbenzyl, furanyl, pyridyl, phthalimido, thienyl, pyrrolyl, imidazolyl, pyrazolyl, thiazolyl, isothiazolyl, oxazolyl, pyrrolidinyl, pyrrolinyl, imidazolidinyl, imidazolinyl, pyrazolidinyl, tetrahydrofuranyl, pyranyl, pyronyl, pyrazinyl, pyridazinyl, piperidinyl, piperazinyl, morpholinyl, thionaphthyl, benzofuranyl, isobenzofuryl, indolyl, oxyindolyl, isoindolyl, indazolyl, indolinyl, azaindolyl, isoindazolyl, benzopyranyl, coumarinyl, isocoumarinyl, quinolyl, isoquinolyl, cinnolinyl, quinazolinyl, pyridopyridyl, benzoxazinyl, quinoxalinyl, chromenyl, chromanyl, isochromanyl, carbolinyl, thiophenyl, thiazolyl, isoxazolyl, isoxazolonyl, isothiazolyl, oxadiazolyl, thiadiazolyl, succinimidyl, maleimidyl, pyridazyl, triazolyl, oxatriazolyl, triazolidinyl, triazolinyl, methoxyltriazolyl, ethoxyltriazolyl, triazolylheptene, triazolyloctene, triazolylnonene, triazolyldecene, methoxyltriazolylheptene, ethoxyltriazolylheptene, methoxyltriazolyloctene, ethoxyltriazolyloctene, methoxyltriazolylnonene, ethoxyltriazolylnonene, methoxyltriazolyldecene, ethoxyltriazolyldecene, methylaminooxomethylsuccinimidethioaminoethanamide, methylaminooxoethylsuccinimidethioaminoethnamide, methylaminooxopropylsuccinimidethioaminoethanamide, methylaminooxomethylsuccinimidethioaminopropanamide, methylaminooxoethylsuccinimidethioaminopropamide, methylaminooxopropylsuccinimidethioaminopropamide, ethylaminooxomethylsuccinimidethioaminoethanamide, ethylaminooxoethylsuccinimidethioaminoethnamide, ethylaminooxopropylsuccinimidethioaminoethanamide, ethylaminooxomethylsuccinimidethioaminopropanamide, ethylaminooxoethylsuccinimidethioaminopropamide, ethylaminooxopropylsuccinimidethioaminopropamide, propylaminooxomethylsuccinimidethioaminoethanamide, propylaminooxoethylsuccinimidethioaminoethnamide, propylaminooxopropylsuccinimidethioaminoethanamide, propylaminooxomethylsuccinimidethioaminopropanamide, propylaminooxoethylsuccinimidethioaminopropamide, propylaminooxopropylsuccinimidethioaminopropamide, methylaminooxomethylmaleimidethioaminoethanamide, methylaminooxoethylmaleimidethioaminoethnamide, methylaminooxopropylmaleimidethioaminoethanamide, methylaminooxomethylmaleimidethioaminopropanamide, methylaminooxoethylmaleimidethioaminopropamide, methylaminooxopropylmaleimidethioaminopropamide, ethylaminooxomethylmaleimidethioaminoethanamide, ethylaminooxoethylmaleimidethioaminoethnamide, ethylaminooxopropylmaleimidethioaminoethanamide, ethylaminooxomethylmaleimidethioaminopropanamide, ethylaminooxoethylmaleimidethioaminopropamide, ethylaminooxopropylmaleimidethioaminopropamide, propylaminooxomethylmaleimidethioaminoethanamide, propylaminooxoethylmaleimidethioaminoethnamide, propylaminooxopropylmaleimidethioaminoethanamide, propylaminooxomethylmaleimidethioaminopropanamide, propylaminooxoethylmaleimidethioaminopropamide, and propylaminooxopropylmaleimidethioaminopropamide,
any of which are optionally substituted with one or more independently selected from =O, COOH, SH, SO₂H, P(OH)(O)₂, halo, trihalomethyl, OH, NH₂, =NH, NH(alkyl), =N(alkyl), NO₂, CN, OCH₃, SO₂NH₂, C(O)H, C(O)CH₃, alkyl, alkoxyalkyl, alkoxyaryl, aralkyl, alkaryl, heterocyclyl, heteroaryl and aryl.

37. The compound, salt, solvate or prodrug of claim 36 wherein X and W are independently selected from the group consisting of: -CH=CH-CH₂-, -C(O)-, -CH₂-, succinimidyl, -(CH₂)₃NH-, -(CH₂)₂C(O)O-, -CH₂C(O)NH-, -(CH₂)₂C(O)NH-,
- (CH₂)₂NHC(O)CH₂CH₂-, -OCH₂-(heteroaryl)-S-(CH₂)₅-C=C-CH₂-,
- (CH₂)₂NHC(O)(CH₂)₂-N-succinimide-S-CH₂CH(NH₂)C(O)NH-,
- (CH₂)₂NHC(O)(CH₂)₂-N-maleimide-S-CH₂CH(NH₂)C(O)NH-,
- (CH₂)₂NHC(O)(CH₂)₂-N-succinimide-S-CH₂CH(NHAc)C(O)NH-, and
- (CH₂)₂NHC(O)(CH₂)₂-N-maleimide-S-CH₂CH(NHAc)C(O)NH-.

38. The compound, salt, solvate or prodrug of claim 37 wherein X and W are independently selected from the group consisting of:
N-(3-ethylamino-3-oxopropyl)-succinimide-thio-2-amino-propan-3-amido, N-(3-ethylamino-3-oxopropyl)-maleimide-thio-2-amino-propan-3-amido, N-(3-ethylamino-3-oxopropyl)-maleimide-thio-2-amino-propan-3-acetamido, {1-[(6E)oct-6-enyl]-1H-1,2,3-triazol-4-yl}methanol, propylamido, propylamino and ethylamido.

39. The compound, salt, solvate or prodrug of claim 26 wherein:
Z and Y are both not present;
p and q are both 0;
K is an ethylene glycol monomer;
B is independently selected from the group consisting of: -C(O)CH₂-, -C(O)- and -C(O)CH₂O-; and
X is independently selected from the group consisting of: N-(3-ethylamino-3-oxopropyl)-succinimide-thio-2-amino-propan-3-amido, N-(3-ethylamino-3-oxopropyl)-maleimide-thio-2-amino-propan-3-amido, {1-[(6E)oct-6-enyl]-1H-1,2,3-triazol-4-yl}methanol, propylamido, and ethylamido.

40. A compound comprising an AT-binding oligosaccharide as defined in any one of the claims 2 to 19, or a salt, solvate or prodrug thereof.

41. A compound comprising an AT-binding oligosaccharide as defined in any one of the claims 1 to 19, conjugated to a linker as defined in any one of the claims 20 to 39, or a salt, solvate or prodrug thereof.

42. The compound, salt, solvate or prodrug of any one of the claims 1 to 39 wherein the protein is a recombinant therapeutic protein.

43. The compound, salt, solvate or prodrug of claim 42 wherein the protein is independently selected from the group consisting of: cytokines, growth factors, hormones, chemokines, antibodies, coagulation factors, apoptotic factors and therapeutic peptides.

44. The compound, salt, solvate or prodrug of any one of the claims 1 to 39 wherein the protein is independently selected from the group: erythropoietin, the interferon family, and enfuvirtide.

45. The compound, salt, solvate or prodrug of claim 44 wherein the protein is interferon-α.

46. The compound, salt, solvate or prodrug of claim 1 of the formula: 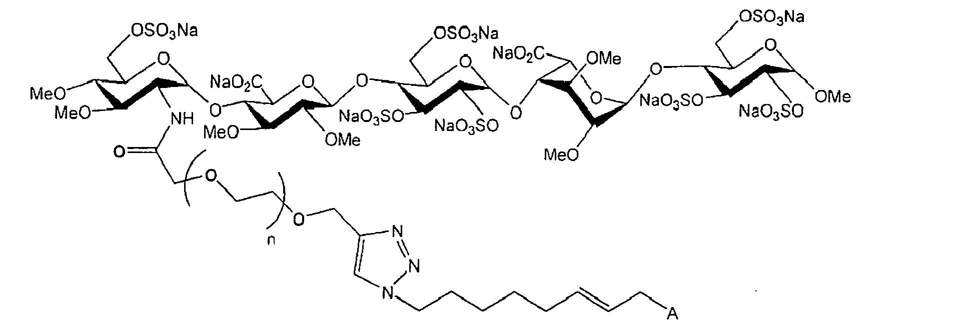 wherein:
A is enfuvirtide; and
n is 6.

47. The compound, salt, solvate or prodrug of claim 1 of the formula: 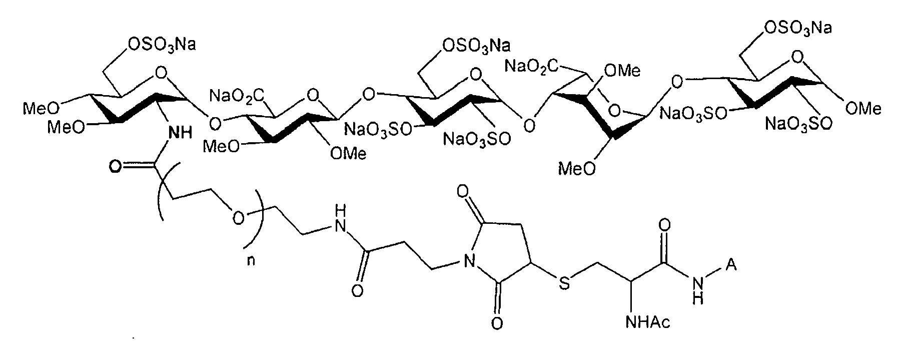 wherein:
A is enfuvirtide; and
n is 12.

48. The compound, salt, solvate or prodrug of claim 1 of the formula: 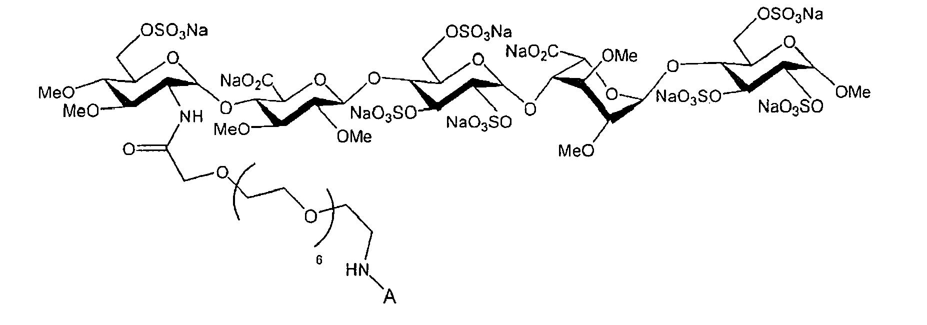 wherein:
A is interferon-α; and
n is 6.

49. The compound, salt, solvate or prodrug of claim 1 of the formula: 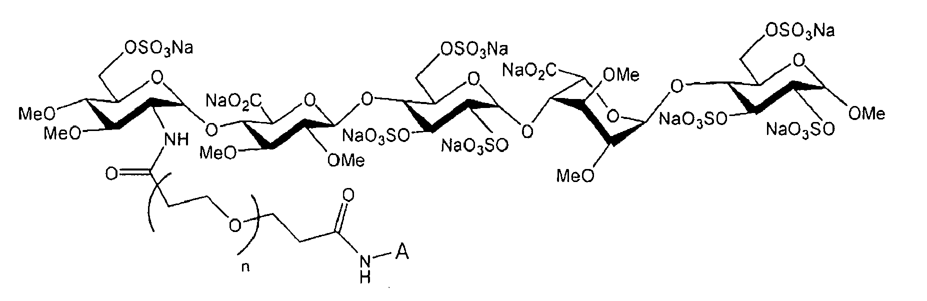 wherein:
A is erythropoietin; and
n is 5.

50. A pharmaceutical composition comprising a compound, salt, solvate or prodrug according to any preceding claim and a pharmaceutically acceptable diluent or carrier.

51. A method of making a pharmaceutical composition according to claim 50, comprising mixing said compound, salt, solvate or pro-drug with a pharmaceutically acceptable diluent or carrier.

52. A compound salt, solvate or prodrug according to any of claims 1 to 49, for use in therapy.

53. The use of an AT-binding oligosaccharide as defined in any one of the claims 1 to 19 or a salt, solvate or prodrug thereof for conjugation to a protein, *via* a linker, to increase the biological half-life of the protein.

54. The use of an AT-binding oligosaccharide as defined in any one of the claims 1 to 19 conjugated to a linker as defined in any one of the claims 20 to 39, or a salt, solvate or prodrug thereof for conjugation to a protein to increase the biological half-life of the protein.
